# EUROPEAN PATENT SPECIFICATION

(11) **EP 1 630 233 B1**
(45) Date of publication and mention of the grant of the patent: **24.02.2010**
(21) Application number: 04745724.7
(22) Date of filing: 03.06.2004
(51) Int. Cl.: C12N 15/82

(54) **NOVEL VECTOR**
NEUARTIGER VEKTOR
NOUVEAU VECTEUR

(30) Priority: 03.06.2003 JP 2003193227; 09.12.2003 JP 2003436431
(43) Date of publication of application: 01.03.2006
(73) Proprietor: NIPPON PAPER INDUSTRIES CO., LTD., Kita-ku, Tokyo 114-0002 (JP)
(72) Inventor: NANTO, Kazuya c/o Nippon Paper Industries Co., Ltd, Tokyo 114-0002 (JP); WATANABE, Keiko c/o Nippon Paper Industries Co., L, u, Tokyo 114-0002 (JP); EBINUMA, Hiroyasu c/o Nippon Paper Industries Co.,, -ku, Tokyo 114-0002 (JP)
(74) Representative: Ward, David Ian
(86) International application number: PCT/JP2004/008080
(87) International publication number: WO 2004/108935

(56) References cited:
- WO-A-01/00809
- WO-A-97/37012
- WO-A1-00/11155
- WO-A2-01/40492
- WO-A2-01/79471
- WO-A2-02/077246
- JP-A- 2001 218 583
- RUSS A P ET AL: "SELF-DELETING RETROVIRUS VECTORS FOR GENE THERAPY" JOURNAL OF VIROLOGY, THE AMERICAN SOCIETY FOR MICROBIOLOGY, US, vol. 70, no. 8, 1 August 1996 (1996-08-01), pages 4927-4932, XP000644457 ISSN: 0022-538X
- BUNTING MICHAELINE ET AL: "Targeting genes for self-excision in the germ line" GENES AND DEVELOPMENT, COLD SPRING HARBOR LABORATORY PRESS, PLAINVIEW, NY, US, vol. 13, no. 12, 15 June 1999 (1999-06-15), pages 1524-1528, XP002144706 ISSN: 0890-9369
- SILVER DANIEL P ET AL: "Self-excising retroviral vectors encoding the Cre recombinase overcome Cre-mediated cellular toxicity" MOLECULAR CELL, CELL PRESS, CAMBRIDGE, MA, US, vol. 8, no. 1, July 2001 (2001-07), pages 233-243, XP002182221 ISSN: 1097-2765
- VERGUNST A.C. ET AL.: 'Site-specific integration of Agrobacterium T-DNA in Arabidopsis thaliana mediated by Cre recombinase' NULEIC ACIDS RESEARCH vol. 26, no. 11, 1998, pages 2729 - 2734, XP002981450
- OW D.W.: 'Recombinase-directed plant transformation for the post-genomic era' PLANT MOLECULAR BIOLOGY vol. 48, no. 1-2, 2002, pages 183 - 200, XP008007551

### SEQUENCE LISTING

<110> Nippon Paper Industries Co.,Ltd.
<120> Novel Vector
<130> P04889400
<150> JP 2003-193227
   <151> 2003-06-03
<150> JP 2003-436431
   <151> 2003-12-09
<160> 12
<210> 1
   <211> 28
   <212> DNA
   <213> Artificial Sequence
<220>
   <223> primer for PCR
<400> 1
   gctctagagc atgtggaggc taacagtg 28
<210> 2
   <211> 28
   <212> DNA
   <213> Artificial Sequence
<220>
   <223> primer for PCR
<400> 2
   gcgagctctc agtgctctac gtaggccg 28
<210> 3
   <211> 20
   <212> DNA
   <213> Artificial Sequence
<220>
   <223> primer for PCR
<400> 3
   tgaacgaaat agacagatcg 20
<210> 4
   <211> 24
   <212> DNA
   <213> Artificial Sequence
<220>
   <223> primer for PCR
<400> 4
   attcgcaggg gataacgcag gaaa 24
<210> 5
   <211> 20
   <212> DNA
   <213> Artificial Sequence
<220>
   <223> primer for PCR
<400> 5
   cgtctgtcga gaagtttctg 20
<210> 6
   <211> 20
   <212> DNA
   <213> Artificial Sequence
<220>
   <223> primer for PCR
<400> 6
   ctatcggcga gtacttctac 20
<210> 7
   <211> 20
   <212> DNA
   <213> Artificial Sequence
<220>
   <223> primer for PCR
<400> 7
   acaatcccac tatccttcgc 20
<210> 8
   <211> 20
   <212> DNA
   <213> Artificial Sequence
<220>
   <223> primer for PCR
<400> 8
   tgcatcggcg aactgatcgt 20
<210> 9
   <211> 19
   <212> DNA
   <213> Artificial Sequence
<220>
   <223> primer for PCR
<400> 9
   cagcagggga aggggatag 19
<210> 10
   <211> 18
   <212> DNA
   <213> Artificial Sequence
<220>
   <223> primer for PCR
<400> 10
   gatgcaatag gtcagctc 18
<210> 11
   <211> 25
<212> DNA
   <213> Artificial Sequence
<220>
   <223> primer for PCR
<400> 11
   cgttcggttg gcagaagcta tgaaa 25
<210> 12
   <211> 20
   <212> DNA
   <213> Artificial Sequence
<220>
   <223> primer for PCR
<400> 12
   cagaaacttc tcgacagacg 20

## Description

### Technical Field

The present invention relates to a vector for modifying certain genes in plants, animals and fungi.

### Background of the Invention

With remarkable advance in the genetic recombination techniques in recent years, various gene introduction methods have been developed and actually used. By putting the results to practical use, for example, in the field of agriculture, recombinant crops and the like to which a novel character was added, which were conventionally impossible, were produced and cultivated in the field in a large scale.

However, in most cases of the gene introduction methods so far put into practical use, the full length of the desired gene is not introduced, and a part thereof is introduced into the host DNA (DNA of a host cell) under a tom-off state. What is more, two or more of the desired gene are introduced at random positions of the host DNA. However, when two or more of the same gene are present on the same DNA (when some of them are not a full length), expression inhibition of the transgene (gene silencing) is induced (Muskens et al., Plant Mol. Biol., 43: 243-260 (2000), Matzke et al., Plant Mol. Biol., 43: 401-415 (2000)). In addition, expression of a gene is greatly influenced by the position on the host DNA into which the gene is introduced (Meyer, Plant Mol. Biol., 43: 221-234 (2000)). Thus, expression pattern and expression quantity of the desired gene introduced by a method so far put into practical use greatly differ respectively, even in the case of recombinants produced from the same gene introduction-treated tissue, wherein introduction of the desired gene was confirmed by DNA analysis and the like.

Accordingly, in order to obtain a recombinant which shows the expression pattern of the desired gene, has its high expression quantity and is stable, by the conventional gene introduction method, in general, it is necessary to select a recombinant in which one copy of a gene is introduced into a position having high expression quantity, from recombinants in which the gene was introduced at random on the chromosome, by producing a large number of recombinants per one desired gene, and carrying out detailed DNA analyses on respective lines obtained by their clonal propagation. Actually, in producing a recombinant crop to which a novel practical character was added by introducing a desired gene, hundreds of independent recombinant lines are produced and their screening is carried out.

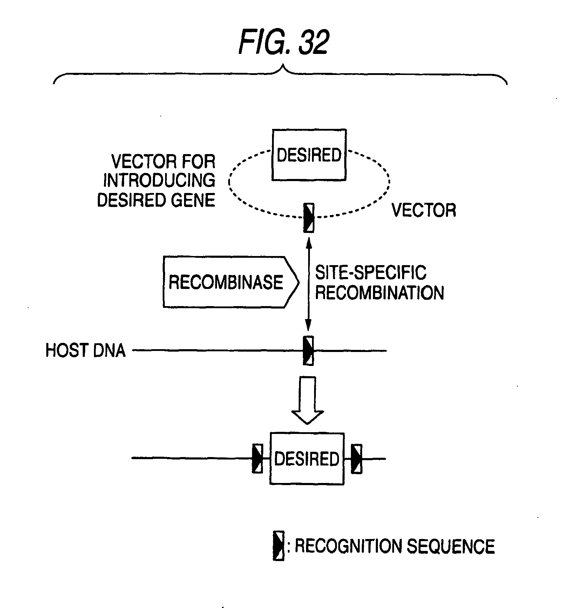

Thus, as a method for introducing one copy of a desired gene to a predetermined position of a host DNA, application of a site-specific recombination system has been examined. That is, a method in which a desired gene is introduced into an adjacent part of recognition sequences being present on a host DNA, making use of a site-specific recombination reaction that occurs between a recognition sequence on the host DNA and a recognition sequence on a vector, in the presence of a site-specific recombinase capable of acting by recognizing these recognition sequences (hereinafter also simply referred to as recombinase), by introducing a cyclic DNA having the desired gene and a recognition sequence, as a vector, into a host cell having a site-specific recombinase recognition sequence (hereinafter also simply referred to as recognition sequence) introduced in advance into the DNA (Fig. 32).

In this method which uses a site-specific recombination system, expression control of a recombinase becomes a big problem. This is because the site-specific recombination reaction is catalyzed by the recombinase both in the introduction direction and the cut out direction of a desired gene, so that it is considerably difficult to obtain a recombinant of interest due to unstable introduction condition of the desired gene. Accordingly, development of a technique for suitably controlling expression of the recombinase sufficient for the introduction of the desired gene and expression shut off of the recombinase after the introduction becomes essential for putting this method into practical use.

For the purpose of solving such problems, a transient method which is carried out by separately preparing a vector having a desired gene and a recognition sequence and another vector having a recombinase, and introducing these two vectors into a host cell and the method of Vergunst *et al*. which can constantly express a recombinase in a host cell and can shut off expression of the recombinase by introducing a desired gene into a target position have so far been proposed (Vergunst et al., Nucl. Acids Res., 26: 2729-2734 (1998)), and a case of succeeding in integrating a desired gene into a target position on the chromosome of a plant by using site-specific recombination systems of phage and yeast (Cre-lox, FLP-FRT), based on these method, has also been reported (review: Ow, Currr. Opin. Biotechnol., 7: 181-186 (1996), Plant Mol. Biol., 48: 183-200 (2002)).

However, even by the transient method, probability of obtaining the intended recombinant is considerably low, and though there is a report stating that recombinants were obtained at a high frequency, its actual proof is still insufficient. On the other hand, according to the method of Vergunst *et al*., the recombinase is continuously expressed until the desired gene is introduced into a target position, so that there is a danger of causing in-stabilization of the chromosome due to generation of an unexpected recombination reaction of the host DNA (Thyagarayan et al., Mol. Cell. Biol., 21: 3926-3934 (2001), Coppoolse et al., Plant Mol. Biol., 21: 3926-3934 (2001)).

Also, in addition to the above-described techniques for introducing a desired gene into a host DNA, a technique in which a gene of a host DNA is deleted or inverted is also included in the genetic recombination techniques. As the method for deleting or inverting a certain gene of a host DNA, a method which uses a site-specific recombination system and transposon in combination has been reported (Van Haaren MJJ, OW DW., Pl. Mol. Biol., 23: 525-533, 1993), but in order to obtain by this method a recombinant in which a predetermined gene is deleted or inverted, it is necessary to produce next generation individual via a crossing step, and what is more, the probability for obtaining the intended recombinant is considerably low. In addition, this method also has a possibility of inducing in-stabilization of chromosome and malformation of recombinant.

Accordingly, the object of the invention is to provide a vector which can introduction one copy of a desired gene to a predetermined position of a host DNA at a high frequency without any bad influences upon the host cell and also can delete or invert the host DNA at a predetermined position without a crossing step and without any bad influences upon the host cell. In addition, another object of the invention is to provide a method for introducing one copy of a desired gene to a predetermined position of a host DNA at a high frequency without any bad influences upon the host cell, which is carried out using this vector, and a method for deleting or inverting a host DNA at a predetermined position without a crossing step and without any bad influences upon the host cell.

### Disclosure

The following (1) to (20) is disclosed:
(1) A vector comprising an introduction cassette inserted between two site-specific recombinase recognition sequences and a site-specific recombinase gene which recognizes the site-specific recombinase recognition sequences, wherein the site-specific recombinase gene is positioned outside the introduction cassette.
(2) The vector according to the above (1), wherein the introduction cassette and the site-specific recombinase gene are positioned such that they can be removed.
(3) The vector according to the above (1), wherein a lethal induction gene or a morphological abnormality induction gene is further positioned outside the introduction cassette.
(4) The vector according to the above (3), wherein the introduction cassette, the site-specific recombinase gene and the lethal induction gene or the morphological abnormality induction gene are positioned such that they can be removed.
(5) The vector according to the above (3), wherein the morphological abnormality induction gene is a plant hormone-related gene.
(6) The vector according to the above (5), wherein the plant hormone-related gene is a cytokinin synthesis gene.
(7) The vector according to the above (1), wherein a desired gene is positioned inside the introduction cassette.
(8) The vector according to the above (1), wherein a part or the whole of a selectable marker gene is positioned inside the introduction cassette.
(9) The vector according to the above (8), wherein at least a promoter moiety of the selectable marker gene is positioned inside the introduction cassette.
(10) The vector according to the above (8), wherein at least a structural gene moiety of the selectable marker gene is positioned inside the introduction cassette.
(11) A method for introducing a gene, which comprises introducing a vector comprising an introduction cassette inserted between two site-specific recombinase recognition sequences and a site-specific recombinase gene which is positioned outside the introduction cassette and recognizes the site-specific recombinase recognition sequences, into a host cell having a DNA in which one site-specific recombinase recognition sequence is present; and introducing the introduction cassette into the host DNA by a site-specific recombination between the introduction cassette moiety of the vector and the one site-specific recombinase recognition sequence on the host DNA.
(12) The method for introducing a gene into a plant according to the above (11), wherein the host is a plant.
(13) A method for introducing a gene, which comprises introducing a vector comprising an introduction cassette inserted between two site-specific recombinase recognition sequences and a site-specific recombinase gene which is positioned outside the introduction cassette and recognizes the site-specific recombinase recognition sequences, into a host cell having a DNA in which a moiety inserted between two site-specific recombinase recognition sequences is present; and introducing the introduction cassette into the host DNA by a site-specific recombination between the introduction cassette moiety of the vector and the moiety inserted between the two site-specific recombinase recognition sequences on the host DNA.
(14) The method for introducing a gene into a plant according to the above (13), wherein the host is a plant.
(15) A method for producing a plant tissue or a plant individual into which a desired gene is introduced, which comprising the following steps (A) to (C):
   (A) introducing a vector comprising an introduction cassette inserted between two site-specific recombinase recognition sequences, and comprising a desired gene inside the introduction cassette and a site-specific recombinase gene which is positioned outside the introduction cassette and recognizes the site-specific recombinase recognition sequences, into a plant cell having a DNA in which one site-specific recombinase recognition sequence is present or a DNA in which a moiety inserted between two site-specific recombinase recognition sequences is present,
   (B) culturing the plant cell into which the vector is introduced in (A), and selecting a plant cell in which a desired gene is introduced into a target position in the DNA by using, as an index, expression of a specific character or disappearance of a specific character observed during the culturing, and
   (C) culturing the plant cell selected in (B) to redifferentiate a plant tissue or a plant individual.
(16) A method for modifying a DNA, which comprises introducing a vector comprising an introduction cassette inserted between two site-specific recombinase recognition sequences and a site-specific recombinase gene which is positioned outside the introduction cassette and recognizes the site-specific recombinase recognition sequences, into a host cell having a DNA in which one site-specific recombinase recognition sequence is present; and deleting or inverting a part of the host DNA by a site-specific recombination between the introduction cassette moiety of the vector and the one site-specific recombinase recognition sequence on the host DNA.
(17) The method for introducing a gene into a plant according to the above (16), wherein the host is a plant.
(18) A method for modifying a DNA, which comprises introducing a vector comprising an introduction cassette inserted between two site-specific recombinase recognition sequences and a site-specific recombinase gene which is positioned outside the introduction cassette and recognizes the site-specific recombinase recognition sequences, into a host cell having a DNA in which a moiety inserted between two site-specific recombinase recognition sequences is present; and deleting or inverting a part of the host DNA by a site-specific recombination between the introduction cassette moiety of the vector and the moiety inserted between the two site-specific recombinase recognition sequences on the host DNA.
(19) The method for modifying a plant DNA according to the above (18), wherein the host is a plant.
(20) A method for producing a plant tissue or a plant individual in which a part of a DNA is deleted or inverted, which comprises the following steps (A) to (C):
   (A) introducing a vector comprising an introduction cassette inserted between two site-specific recombinase recognition sequences, and a site-specific recombinase gene which is positioned outside the introduction cassette and recognizes the site-specific recombinase recognition sequences into a plant cell having a DNA in which one site-specific recombinase recognition sequence is present or a DNA in which a moiety inserted between two site-specific recombinase recognition sequences is present,
   (B) culturing the plant cell into which the vector in (A) is introduced in (A), and selecting a plant cell in which a DNA on a target position is deleted or inverted by using, as an index, expression of a specific character or disappearance of a specific character observed during the culturing, and
   (C) culturing the plant cell selected in (B) to redifferentiate a plant tissue or a plant individual.

### Brief Description of the Drawings

Fig. 1 is a graph showing an example of the introduction cassette to be used in the vector of the invention.
Fig. 2 is a drawing showing, among the vectors of the invention, an example of the basic structure of a removal type vector and its function.
Fig. 3 is a drawing showing, among the vectors of the invention, another example of the basic structure of a removal type vector and its function.
Fig. 4 is a drawing showing a mechanism in which a desired gene is introduced into a predetermined position of the host DNA by the vector of the invention.
Fig. 5 is a drawing showing another mechanism in which a desired gene is introduced into a predetermined position of the host DNA by the vector of the invention.
Fig. 6 is a drawing showing still another mechanism in which a desired gene is introduced into a predetermined position of a host DNA by the vector of the invention.
Fig. 7 is a drawing showing a mechanism in which the vector of the invention deletes a host DNA from a predetermined position.
Fig. 8 is a drawing showing another mechanism in which the vector of the invention deletes a host DNA from a predetermined position.
Fig. 9 is a drawing showing effect of a lethal induction gene in the vector of the invention.
Fig. 10 is a drawing showing effect of a morphological abnormality induction gene in the vector of the invention.
Fig. 11 is a drawing showing behavior of a morphological abnormality induction gene when the vector of the invention acts in such a manner that the host DNA is deleted at a predetermined position.
Fig. 12 is a drawing showing a mechanism in which a desired gene is introduced into a predetermined position of the host DNA when a removal type vector among the vectors of the invention is used.
Fig. 13 is a drawing showing a case in which a desired gene is introduced into a target position of the host DNA using the vector of the invention into which a part of a selectable marker gene is introduced.
Fig. 14 is a drawing showing a desired gene introducing mechanism in Example 1 or 3 of the invention.
Fig. 15 is a drawing showing a construction scheme of a vector pTLGUSAmpcodN for recognition sequence introduction.
Fig. 16 is a drawing showing a construction scheme of the vector p2nd 1 of the invention.
Fig. 17 is a drawing showing a gene introduction mechanism when a desired gene is introduced by p2nd1 into a host DNA into which a recognition sequence is introduced by pTLGUSAmpcodN.
Fig. 18 is a drawing showing a relationship between the probe or primer used in the example of the invention and a host DNA into which pTLGUSAmpcodN is introduced.
Fig. 19 is a drawing showing a relationship between the probe or primer used in Example 1 of the invention and a host DNA, which is hypothesized when a desired gene is introduced by a site-specific recombination between a recognition sequence introduced by pTLGUSAmpcodN and p2nd 1.
Fig. 20 is a drawing (photograph) showing a result of Southern analysis on the recombinant strains 2-2-14, 2-2-20, 2-2-27, 2-2-28 and 2-2-29 obtained in Example 1.
Fig. 21 is a drawing showing a construction scheme of the vector p2ndΔRSipt of the invention.
Fig. 22 is a drawing showing a gene introduction mechanism when a desired gene is introduced by p2ndΔRSipt to a host DNA into which a recognition sequence is introduced by pTLGUSAmpcodN.
Fig. 23 is a drawing showing a relationship between the probe or primer used in Example 2 of the invention and a host DNA, which is hypothesized when a desired gene is introduced by a site-specific recombination between a recognition sequence introduced by pTLGUSAmpcodN and p2ndΔRSipt.
Fig. 24 is a drawing (photograph) showing a result of Southern analysis on a recombinant strain 91-1 obtained in Example 2.
Fig. 25 is a drawing showing a construction scheme of the vector p2nd20 of the invention.
Fig. 26 is a drawing showing a gene introduction mechanism when a desired gene is introduced by p2nd20 to a host DNA into which a recognition sequence is introduced by pTLGUSAmpcodN.
Fig. 27 is a drawing showing a mechanism when a host DNA into which a recognition sequence is introduced by pTLGUSAmpcodN is deleted at a predetermined position using p2nd20.
Fig. 28 is a drawing showing a relationship between the probe or primer used in Example 3 of the invention and a host DNA, which is hypothesized when a desired gene is introduced by a site-specific recombination between a recognition sequence introduced by pTLGUSAmpcodN and p2nd20.
Fig. 29 is a drawing (photograph) showing a result of Southern analysis on recombinant strains 12-1 and 12-2 obtained in Example 3.
Fig. 30 is a drawing (photograph) showing a result of Southern analysis on a gene-deleted individual strain 17-1 obtained in Example 3.
Fig. 31 is a drawing showing a relationship between the probe or primer used in Example 3 of the invention and a host DNA, which is hypothesized when a part of DNA is deleted by a site-specific recombination between a recognition sequence introduced by pTLGUSAmpcodN and p2nd20.
Fig. 32 is a drawing showing a gene introduction method by a conventional type vector, which uses a site-specific recombination system.

### Best Mode for Carrying Out the Invention

The invention is described below in detail.

A site-specific recombinase recognition sequence and a site-specific recombinase gene are elements of a site-specific recombination system. As the site-specific recombination system, for example, Cre/lox system, R/RS system, FLP/FRT system, *cer* system, *fim* system and the like which are separated from microorganisms such as phages, bacteria (e.g., *Escherichia coli*) and yeasts are known (as a review, N. L. Craig, Annu. Rev. Genet., 22: 17, 1988), but its presence in higher organisms is not known yet. However, it has been revealed that even when these site-specific recombination systems separated from these microorganisms are introduced into biotic kind different from the original biotic kind, namely when introduced into plants or animals, they behaves similarly to the behavior in the original organisms.

The recognition sequence and recombinase gene to be used in the invention can be used by freely selecting from combinations of recognition sequences and recombinase genes constituting such site-specific recombination systems. Especially, Cre/lox system and R/RS system are desirable as the site-specific recombination system to be used in the invention, because they are broadly used in animals, plants and the like and have high recombination efficiency. Of course, it is possible to use a mutation type recognition sequence in which the DNA sequence of a wild type recognition sequence is partially modified artificially or spontaneously. However, it is necessary that the recognition sequence in this case can be recognized by the recombinase encoded by the recombinase gene which is present on the vector of the invention together with this.

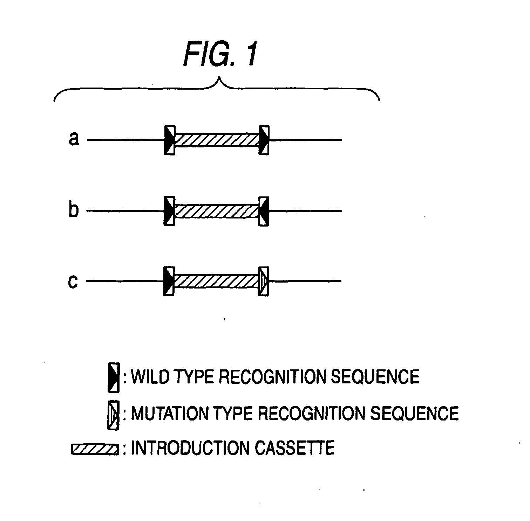

The introduction cassette in the vector of the invention is a moiety inserted between the two recognition sequences where the recombinase gene is not present. Direction of the two recognition sequences inserting this introduction cassette may be either the same direction or opposite directions. In addition, it is possible to use a mutation type recognition sequence as either one of the two recognition sequences or as both of the recognition sequences (Fig. 1).

When two wild type recognition sequences or two mutation type recognition sequences are mutually the same kind and facing the same direction, this introduction cassette is removed from the vector due to the presence of a recombinase which acts by recognizing these recognition sequences. In addition, when two wild type recognition sequences or two mutation type recognition sequences are mutually the same kind but facing opposite directions, or two recognition sequences are mutually different (when one is a wild type recognition sequence and the other is a mutation type recognition sequence, or when they are different kinds of wild type recognition sequences or mutation type recognition sequences), this introduction cassette is not removed from the vector even in the presence of a recombinase which acts by recognizing these recognition sequences. Also, it is known that the site-specific recombination system to be used in the invention functions disregard of the length of the region which induces recombination, and a case in which a DNA of 230 kb matching the length of chromosome was introduced and a case in which an operation of a DNA of Mb level was carried out using a yeast have also been reported. Accordingly, the length of the introduction cassette is not particularly limited in the invention. In addition, the position of the recombinase gene to this introduction cassette is also not particularly limited. The object of the invention can be attained so far as it uses a vector which has an introduction cassette inserted between two site-specific recombinase recognition sequences and a site-specific recombinase gene encoding a site-specific recombinase capable of functioning by recognizing these recognition sequences, wherein this recombinase gene is positioned outside the introduction cassette.

In the vector of the invention, a recombinant in which a desired gene is introduced into a predetermined position of its host DNA, or a recombinant in which the host DNA is deleted or inverted at a predetermined position, can be securely obtained by positioning a lethal induction gene or a morphological abnormality induction gene outside the introduction cassette, together with the recombinase gene.

Also, the lethal induction gene means the whole gene encoding a protein capable of spoiling function of cells to thereby cause death of the cells. As such a lethal induction gene, for example, genes encoding RNase, DAM methylase, cytosine deaminase (codA), diphtheria toxin, Bax and the like are generally known. However, according to the invention, genes other than these can also be used regardless of their kinds so long as they are lethal induction genes.

Also, the morphological abnormality induction gene means the whole gene capable of causing differentiation into unusual forms by putting the direction of propagation and differentiation of host cells out of order by its expression. As such a morphological abnormality induction gene, in the case of plants, for example, plant hormone-related genes which cause dwarfing such as a plant hormone synthesis system gene and a plant hormone signal transduction system gene, collapse of apical dominance, change of pigment, crown gall, hairy root, waving of leaf and the like in the host plants can be used.

In this case, the plant hormone synthesis system gene means a gene encoding a protein related to the synthesis of a plant hormone or the like, and for example, *ipt* (isopentenyl transferase) gene (A.C. Smigocki and L.G. Owens, Proc. Natl. Acad. Sci. USA, 85: 5131 (1988)), *iaaM* (tryptophan monooxygenase) gene (H.J. Klee et al., GENES & DEVELOPMENT, 1: 86 (1987)), gene *5* gene (H. Kerber et al., EMBO Journal, 10: 3983 (1991)), gene *6b* gene (P.J.J. Hooyaas et al., Plant Mol. Biol., 11: 791 (1988)) and a *rol* gene group of *rolA* to *D* (F.F. White et al., J. Bacteriol., 164: 33 (1985)) which are present in plant pathogens such as *Agrobacterium,* as well as *iaaL* (indoleacetic acid-lysine synthetase) gene which is present in a subspecies of *Pseudomonas syringae* (*Pseudomonas syringae* subsp. *savastanoi*) (A. Spena et al., Mol. Gen. Genet., 227: 205 (1991)) and also homeobox genes, phytochrome genes and the like in various plants are known.

On the other hand, the plant hormone signal transduction system gene means genes encoding proteins having a sensor function to recognize the presence of plant hormones such as gibberellin, ethylene, auxin and cytokinin and proteins concerned in a series of signal transduction pathway for introducing signals from the sensor, and, for example, an ethylene receptor gene *ETR1* gene (C. Chang et al., Science, 262: 539 (1993)), *CK11* gene considered to be a cytokinin receptor gene (T. Kakimoto, Science, 274: 982 (1996)) and its mutant (ex. *CK12* gene) and *GCR1* gene (S. Plakidou-Dymock et al., Current Biology, 8: 315 (1998)), as well as *IBC6* gene and *IBC7* gene (I. Brandstatter and J.J. Kieber, The Plant Cell, 10: 1009 (1998)) and the like are known.

When the invention is applied to a plant, any one of these morphological abnormality induction genes can be used, but particularly among them, the *ipt* gene which cause collapse of apical dominance and the *rol* gene group capable of causing formation of hairy roots, and dwarfing, waving of leaves and the like in a plant regenerated from hairy roots, are desirable as the morphological abnormality induction gene when the invention is applied to a plant, because they cause characteristic abnormal morphology and prevent re-differentiation of plant individuals.

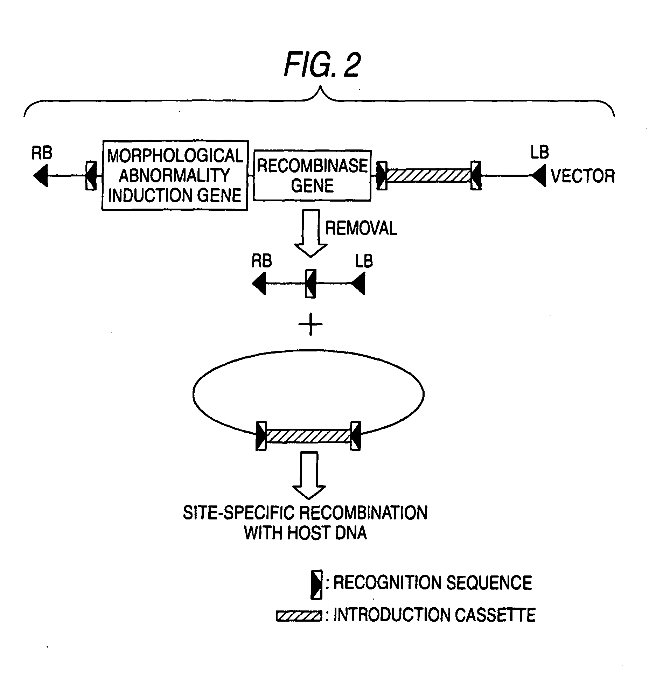

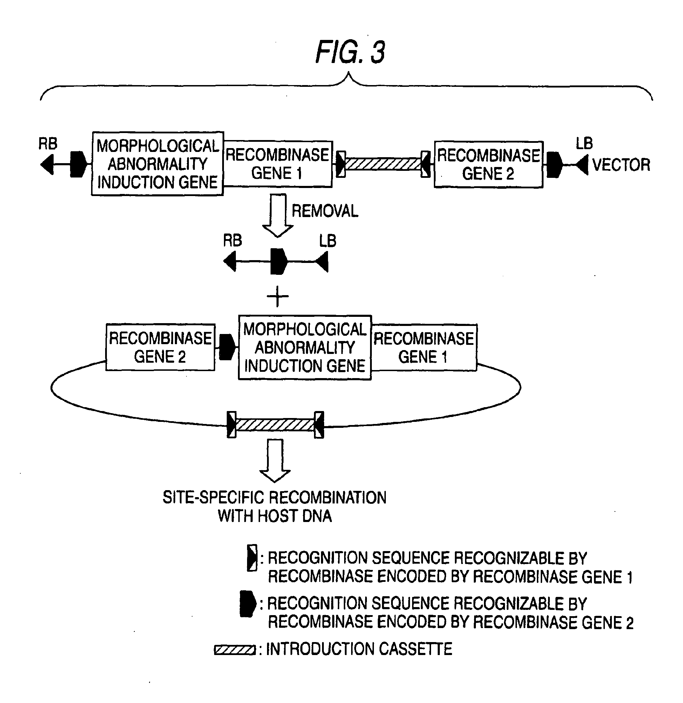

In addition, according to the invention, the introduction cassette and the recombinant gene, or the introduction cassette, the recombinase gene and the lethal induction gene or the morphological abnormality induction gene may be positioned such that they can be removed (hereinafter such a type of the vector of the invention is called removal type vector). This can also be realized by, for example, inserting the introduction cassette and recombinase gene between either one of the recognition sequences inserting the introduction cassette and a recognition sequence of the same kind and the same direction (Fig. 2), or using recognition sequences of kind different from the recognition sequences inserting the introduction cassette, and inserting a recombinase which recognizes these different kinds of recognition sequences between these two recognition sequences facing the same direction, together with the recognition cassette, the recombinase gene which recognizes the recognition sequences inserting the introduction cassette and the like (Fig. 3). The removal type vector is particularly effective when a gene is introduced into plant cells by the *Agrobacterium* method using a binary vector which is apt to cause nonspecific introduction. Also, either of wild type and mutation type can be used also as the recognition sequences to be used in the above-described case.

In the vector of the invention, when one copy of a desired gene is positioned inside the introduction cassette, one copy of the desired gene can be introduced at a high frequency into a predetermined position of the host DNA. In this case, as the desired gene, various kind can be selected according to the object, such as a gene capable of adding a superior character to a host organism and a gene which is not always capable of adding a superior character to a host organism but is necessary for study on a gene expression mechanism.

In addition, when a selectable marker gene is used in the invention, a recombinant in which a desired gene is introduced into a predetermined position of a host DNA and a recombinant in which the host DNA is deleted or inverted at a predetermined position can be efficiently selected, using its expression or disappearance of its expression as the index. The position and the number of the selectable marker gene are not particularly limited. For example, the selectable marker gene may be positioned inside the introduction cassette or outside it. Also, it may be positioned at a position of the host DNA where a desired gene is to be introduced, or a position where the host DNA is to be deleted or inverted (also called target position). In addition, the promoter moiety of the selectable marker gene and other moieties (structural gene, terminator, *etc*.) may be separately positioned inside the introduction cassette and the target position of the host DNA. Also, any kind can be used as the selectable marker gene, so long as it can be introduced into the vector of the invention or the host DNA, and it is expressed in a host cell and the expression or disappearance of the expression can be easily detected. For example, when the host is a plant, antibiotics resistant genes such as a kanamycin resistant (NPTII) gene and a hygromycin resistant (hygromycin phosphotransferase) gene, herbicide resistance genes such as a phosphinothricin acetyltransferase (bar) gene which provides resistance against bialaphos, and the like can be cited as typical examples of such a selectable marker gene. According to the invention, one or more of these selectable marker genes can also be used.

When a desired gene is introduced into a predetermined position of a host DNA using the vector of the invention, firstly, one or two of recognition sequences similar to the recognition sequences inserting the introduction cassette of the vector to be used are inserted in advance into the target position of the host DNA, and then the vector of the invention is introduced into the host cell which keeps this DNA using a conventionally known gene introduction method. On the other hand, when the host DNA is deleted or inverted at a predetermined position, one or two of recognition sequences similar to the recognition sequences inserting the introduction cassette of the vector to be used are inserted into an adjacent region of the target position. Thereafter, the vector of the invention is introduced into the host cell which keeps this DNA, in the same manner as the case of the introduction of a desired gene except for this.

The recognition sequences can be introduced into a host DNA in accordance with a usual method. In this case, the recognition sequences are introduced at random into the host DNA, and the vector of the invention may be introduced by carrying out DNA analysis by a conventionally known method and thereby selecting a host cell in which the predetermined number of recognition sequences are introduced into the target position or an adjacent region of the target position.

As the method for introducing the vector of the invention into a host cell, for example, physical and chemical methods such as a microinjection method, an electroporation method, a polyethylene glycol method, a fusion method and a high speed ballistic penetration method can be used as direct introduction methods into a plant or animal cell (I. Potrykus, Annu. Rev. Plant Physiol. Plant Mol. Biol., 42: 205 (1991)). In addition, for the plant cells, an indirect introduction method which is carried out via a virus or bacterium which infects upon a plant can also be used (I. Potrykus, Annu. Rev. Plant Physiol. Plant Mol. Biol., 42: 205 (1991)). In this case, cauliflower mosaic virus, gemini virus, tobacco mosaic virus, brome mosaic virus and the like can be used as the virus, and *Agrobacterium tumefaciens* (hereinafter referred to as *A. tumefaciens*), *Agrobacterium rhizogenes* and the like can be used as the bacterium.

The vector of the invention introduced into a host cell introduces a desired gene into a predetermined position of a host DNA, or deletes or inverts the host DNA at a predetermined position, by causing recombination between the host DNA through the mechanism described in detail in the following item <Action>. In order to obtain a recombinant tissue or a recombinant cell from the thus DNA-modified host cell, the cell after gene introduction treatment carried out using the vector of the invention is proliferated and re-differentiated in the usual way, while carrying out selection by optionally using an index such as expression of a specific character by a desired gene or a selectable marker gene or disappearance of the specific character caused by deletion or the like of the gene.

### <Action>

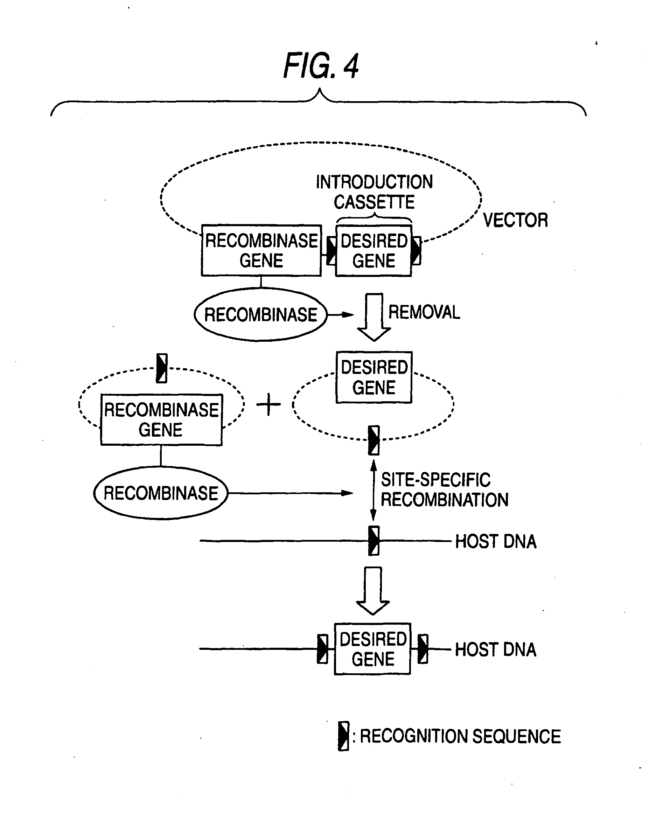

When the vector of the invention is used, a desired gene can be introduced into a predetermined position of a host DNA, or the host DNA can be deleted or inverted at a predetermined position. A mechanism in which a gene is introduced into a predetermined position and a mechanism in which deletion or inversion is generated at a predetermined position, induced by a recombination between the vector of the invention introduced into a host cell and the host DNA, are described in the following based on the examples shown in Figs. 4 to 14. However, the gene introduction mechanism and DNA modification mechanism by the vector of the invention are not limited to these examples.

Firstly, in the case of a vector of the construction of Fig. 4 in which an introduction cassette is inserted between two site-specific recombinase recognition sequences and positioned such that it can be removed, and a desired gene is introduced into the inside of this introduction cassette, and a recombinase gene encoding a site-specific recombinase capable of performing its action by recognizing these recognition sequences is introduced into the outside thereof, when this is introduced into a host cell, the introduction cassette moiety is removed and cut out in a cyclic form by the action of the recombinase expressed from the recombinase gene introduced into this vector. In this case, when one of recognition sequences similar to the recognition sequences inserting this introduction cassette is introduced into a DNA contained in the host cell, the above-described vector-derived recombinase again functions to induce site-specific recombination of the cyclic form introduction cassette moiety and the recognition sequences on the host DNA, and the desired gene is introduced into the position of host DNA into which the recognition sequence is introduced (Fig. 4).

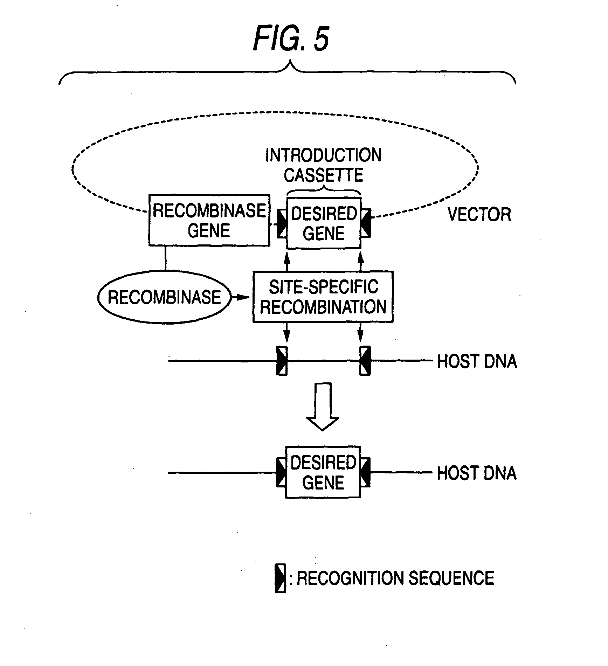

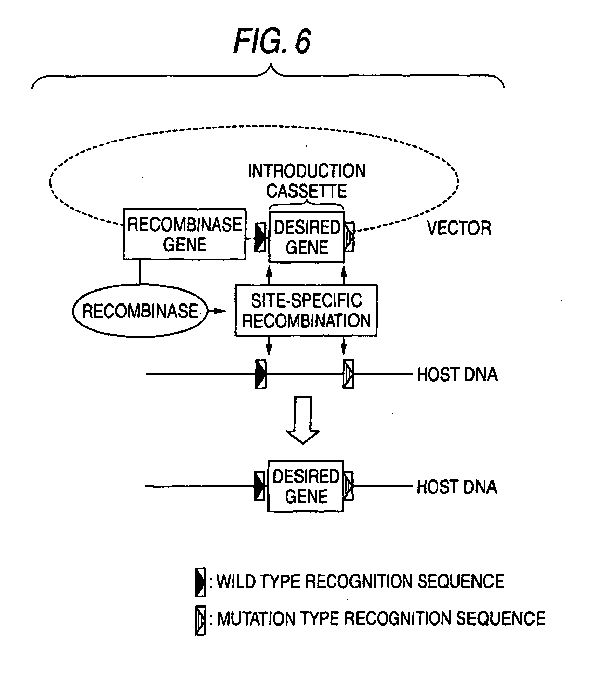

On the other hand, when a vector like Fig. 5 or 6 in which the constructions regarding the recombinase gene and the desired gene are similar to that of the vector of Fig. 4, but the introduction cassette is positioned such that it cannot be removed, is introduced into a host cell, and when a moiety inserted between the two recognition sequences respectively having the same kind and the same direction as the recognition sequences inserting the introduction cassette is present, site-specific recombination is caused between the introduction cassette and this moiety, and the desired gene is introduced into the region which is inserted between the recognition sequences of the host DNA (Figs. 5 and 6).

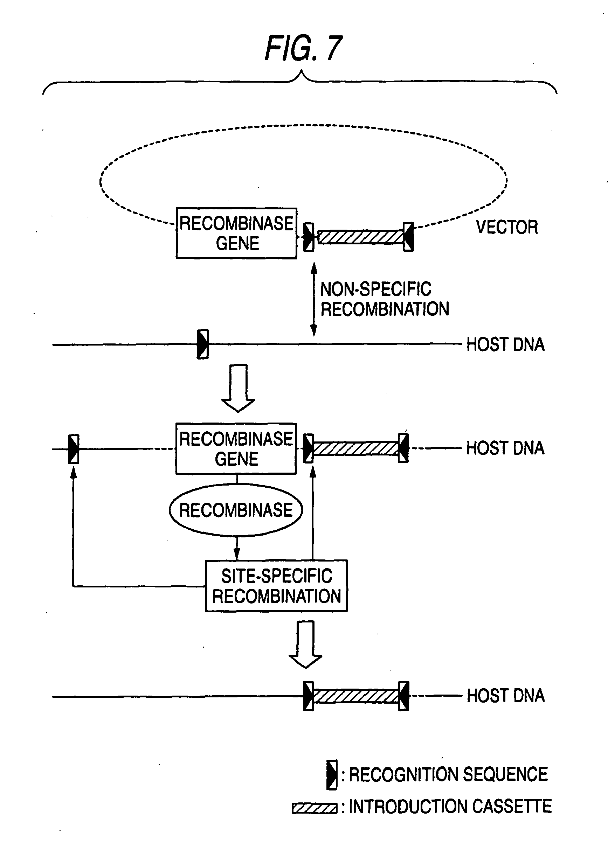

Also, when the vector shown in Fig. 5 or 6 is introduced into a host cell having a DNA in which one or more of recognition sequences similar to the recognition sequences inserting this introduction cassette is introduced, the introduction cassette and recombinase gene of the vector are non-specifically introduced into the DNA, thus causing a phenomenon in which a space between the recognition sequences inserting the introduction cassette and the recognition sequences which is introduced into the host cell is removed by the action of the recombinase expressed from this recombinase gene. Accordingly, when one or more of recognition sequences similar to the recognition sequences inserting the introduction cassette are introduced into an adjacent region of the target position in the host DNA, the DNA existing on the target position can be deleted (Fig. 7). In this case, it is not necessary to position a desired gene on the inside of the introduction cassette.

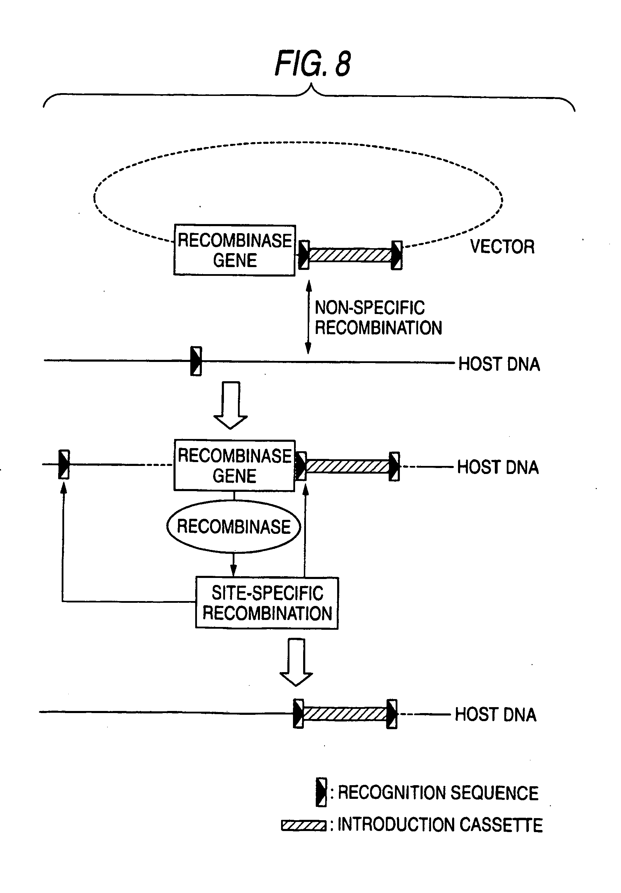

In addition, the above-described non-specific introduction into host DNA is slightly caused, even in the vector of Fig. 4 in which the introduction cassette is positioned such that it can be removed. Accordingly, also in this case, when one of recognition sequences similar to the recognition sequences inserting the introduction cassette in present in the host DNA, it is possible that the DNA existing in its adjacent region is deleted (Fig. 8). However, it is considered that its frequency is markedly low.

When a desired gene is introduced into a predetermined position of a host DNA or when the host DNA is deleted at a predetermined position in the cases shown in Fig. 4 to Fig. 8, there is a case in which the vector of the invention introduced into a host cell causes nonspecific recombination with a DNA which is different from the DNA wherein the target position is present so that the recombinase gene is introduced into the DNA which is different from the DNA wherein the target position is present. In addition, there is a case in which recombination is caused between a host DNA and two or more vectors so that, even when it is possible to introduce a desired gene into a predetermined position or to cause deletion at a predetermined position, the above-described unintended introduction of the recombinase gene is caused at the same time. In such a case, the recombinase gene is not removed from the host DNA but continues to express, so that the host DNA is in-stabilized.

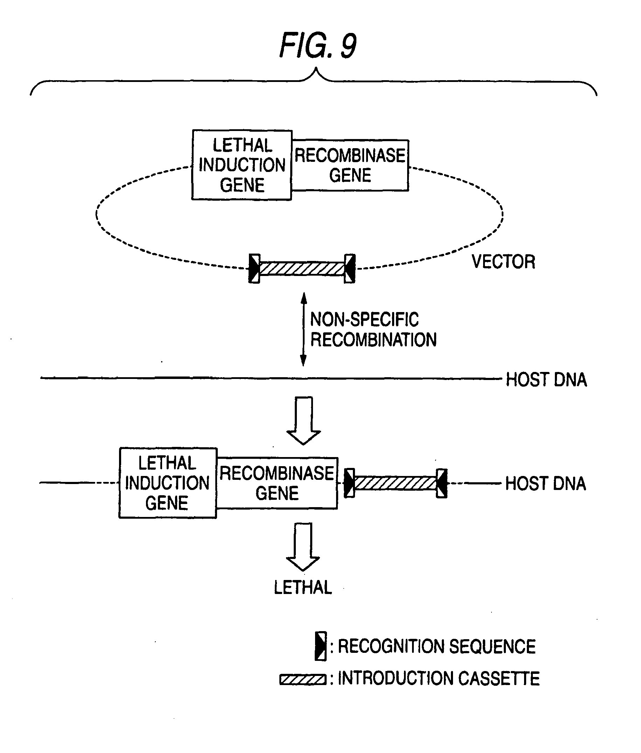

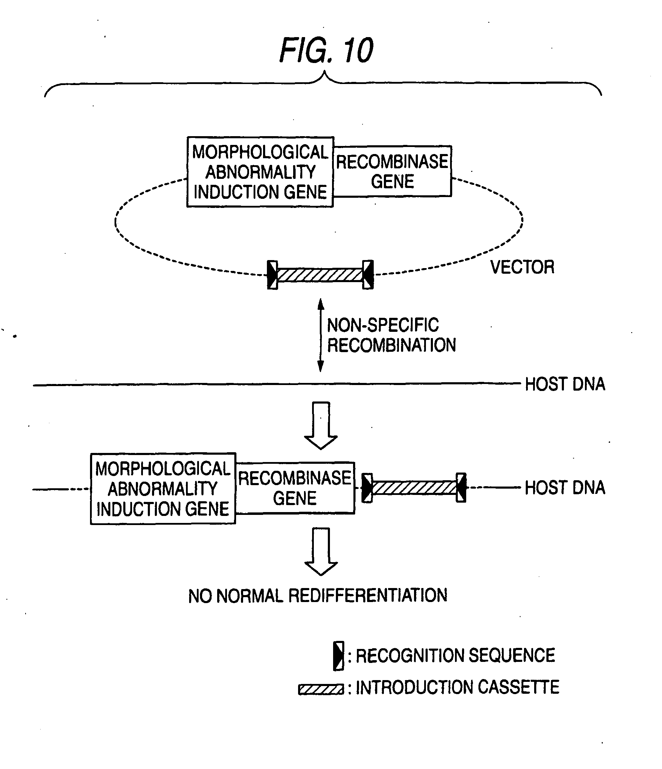

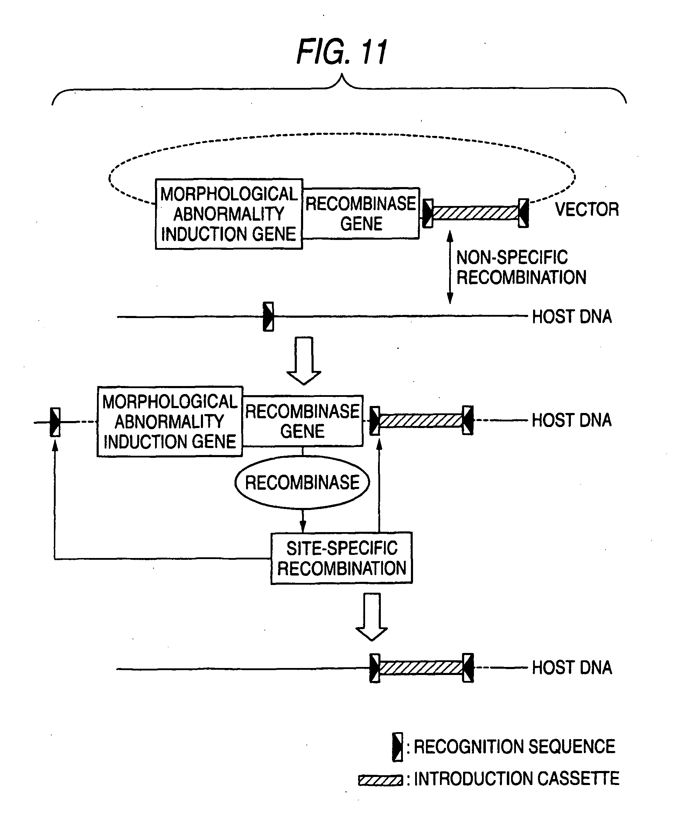

Figs. 9 and 10 show examples in which a lethal induction gene or a morphological abnormality induction gene is positioned, together with a recombinase gene, outside the introduction cassette of the vector of the invention. Like these drawings, which a lethal induction gene or a morphological abnormality induction gene is positioned, together with a recombinase gene, outside the introduction cassette, the recombinant gene is not removed from the host DNA due to unintended introduction, and when introduced for continuous expression, this lethal induction gene or morphological abnormality induction gene also continues its expression, so that the host cell which keeps the DNA wherein such a recombination us caused results in death or causes abnormal morphology such that a normal recombinant cannot be obtained. Accordingly, it becomes possible to preferentially obtain a recombinant derived from a host cell having a DNA in which the desired gene is introduced only to the predetermined position, or a DNA wherein deletion or inversion is caused only at the predetermined position, due to generation of intended introduction. Also, in this case of the DNA in which the desired gene is introduced into the predetermined position, or a DNA wherein deletion or inversion is caused at the predetermined position, due to generation of intended introduction, the lethal induction gene or morphological abnormality induction gene is not introduced into the host DNA or is removed even when introduced (e.g., Fig. 11), so that bad influences are not exerted upon host cells having these DNA moieties.

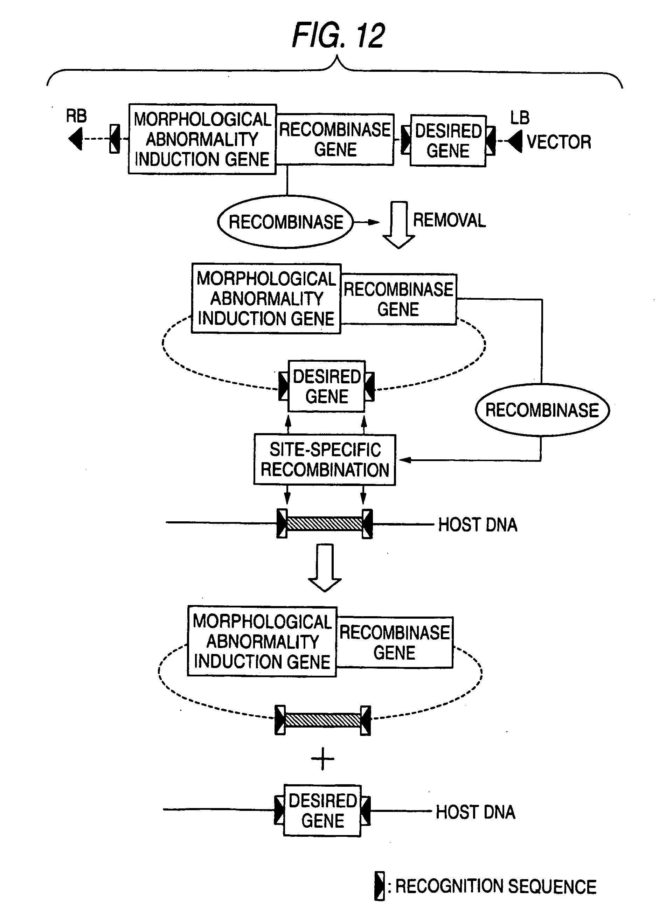

Fig. 12 is an illustration showing an example in which a desired gene is introduced into a predetermined position of a host DNA by a removal type vector. When the removal type vector is introduced into a host cell, the introduction cassette and a recombinase gene and the like are once removed from the vector, and recombination is caused between this removed part and the host DNA, so that the desired gene is introduced into a predetermined position of the host DNA, or deletion or inversion is caused at a predetermined position. The mechanisms after removal of the introduction cassette and a recombinase gene and the like until their recombination with the host DNA are the same as the mechanisms so far described.

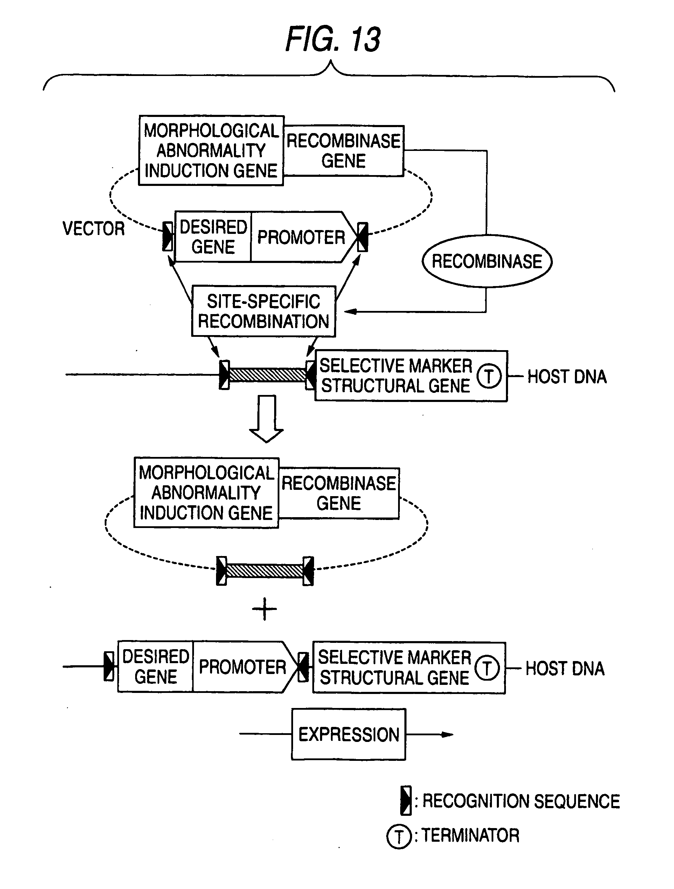

Fig. 13 is an example in which a desired gene is introduced into a target position of a host DNA using a vector in which the introduction cassette is positioned such that it cannot be removed, wherein the desired gene and the promoter moiety of a selectable marker gene are introduced into the inside of the introduction cassette, and a vector having such an introduction cassette is used for a host DNA in which the structural gene and terminator moiety of the above-described selectable marker gene are introduced in advance into an adjacent region of the target position. When introduction of a desired gene is carried out by a combination of a vector having such an introduction cassette with a host DNA, the selectable marker gene is expressed when site-specific recombination is caused between the introduction cassette and a target position of the host DNA, and thereby the desired gene is introduced into this target position, so that a tissue derived from a host cell which keeps the DNA in which the intended recombination is caused can be selected efficiently. Also, contrary to the case of Fig. 13, the structural gene and terminator moiety of the selectable marker gene may be positioned inside the introduction cassette, and the promoter moiety in a region adjacent to the target position of the host DNA, as a matter of course.

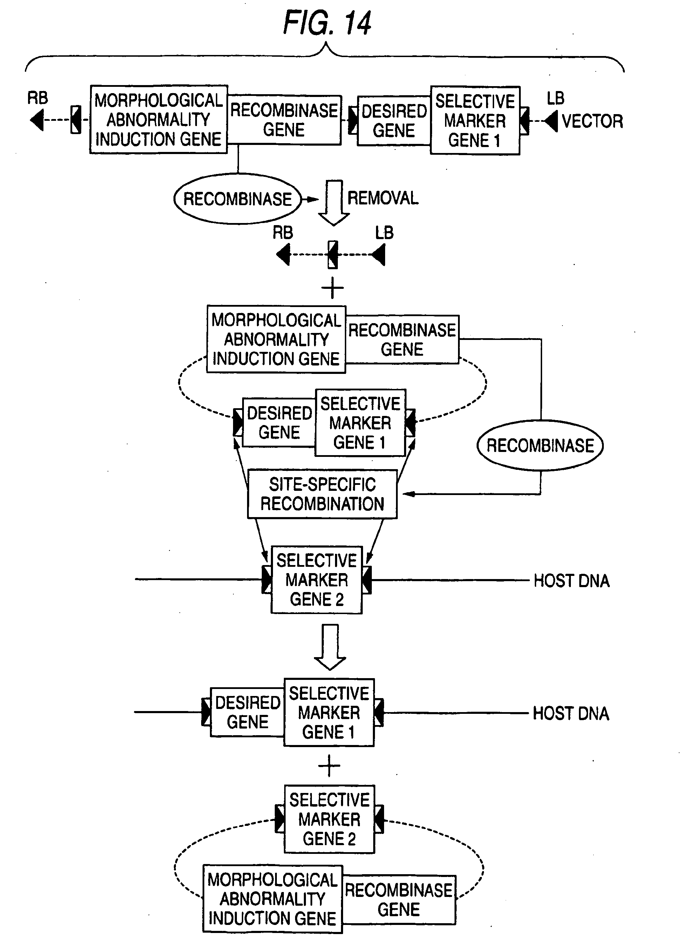

Fig. 14 is a drawing showing a mechanism for introducing a desired gene in Example 1 or 3 of the invention. In Example 1 or 3, one in which the desired gene and a selectable marker gene 1 are introduced into inside an introduction cassette, and a recombinase gene and a morphological abnormality induction gene are introduced into outside the introduction cassette, and the introduction cassette it self is positioned such that it cannot be removed, and the introduction cassette, recombinase gene and morphological abnormality induction gene are positioned such that they can be removed, is used as the vector of the invention, and recombination was carried out by introducing this into a host cell having a DNA in which a selectable marker gene 2 (a gene which shows an expression different from the selectable marker gene 1) is positioned at a target position. In this case, when the desired gene is introduced into the target position of a host DNA, the selectable marker gene 1 is also introduced into the target position of the host DNA and is expressed. On the other hand, since the selectable marker gene 2 is removed from the host DNA and deprived of its function, it is possible to efficiently select a recombinant in which a desired gene is introduced into a predetermined position due to the intended recombination, by selecting a cell after the gene introduction treatment using expression of the selectable marker gene 1 and/or disappearance of the expression of the selectable marker gene 2 as the index.

The invention is described below in detail based on Examples. However, the invention is not limited to Examples described below. Also, in the following Examples, unless otherwise indicated, more detailed test operations were carried out in accordance with Molecular Cloning (Sambrook et al., 1989) or handling instructions of manufacturers.

### Example 1

### I. Construction of vector for recognition sequence introduction to host DNA

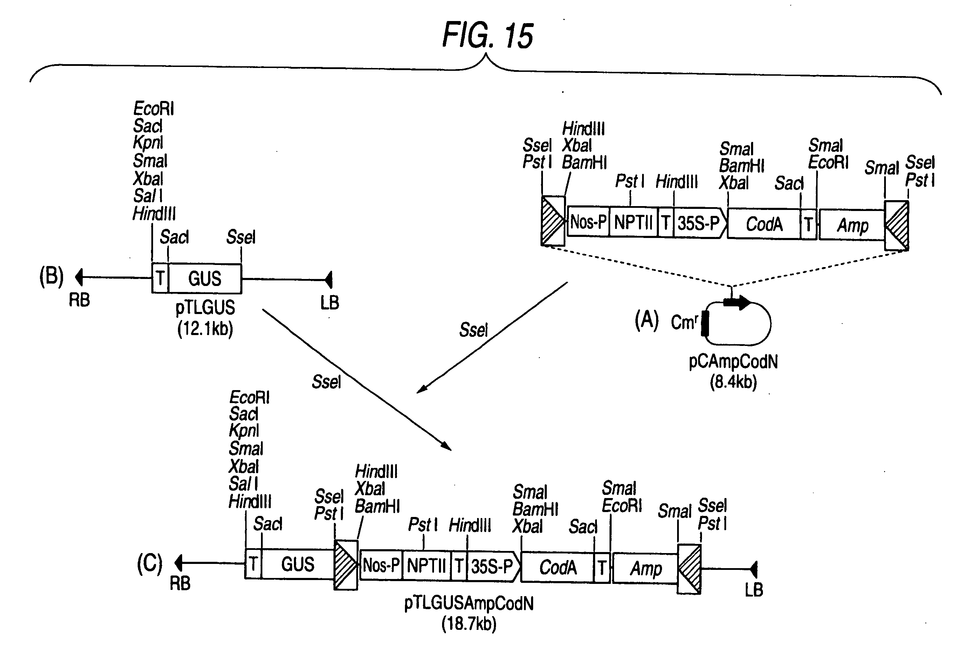

A plasmid pCAmpCodN having a structure in which an NPTII structural gene prepared by ligating the promoter of a nopaline synthase gene (Nos-P) and the polyadenylation signal of nopaline synthase gene (hereinafter, nopaline synthase-derived polyadenylation signal was used in all cases), a codA structural gene prepared by ligating the 35S promoter (35S-P) and the polyadenylation signal, and an ampicillin resistance (Amp) gene were ligated in this order, wherein these are inserted between site-specific recombinase recognition sequences RS mutually facing opposite directions was constructed (Fig. 15A). In this case, the codA structural gene was obtained by the PCR method using genomic DNA of *Escherichia coli* (DH5α, purchased from TOYOBO) as the template, and using a primer-α (5'-gctctagagc atgtggaggc taacagtg-3') represented by SEQ ID NO:1 and a primer-β (5'-gcgagctctc agtgctctac gtaggccg-3') represented by SEQ ID NO:2. Also, in the drawing, the triangle of half-tone dot meshing surrounded by a square frame shows RS and direction of the sequence, and T represents polyadenylation signal (hereinafter the same shall apply).

On the other hand, a plasmid pTLGUS was prepared (Fig. 15B) by ligating a promoter sequence-removed GUS gene to a region between the restriction enzymes *Eco*RI and *Sse*338701 (hereinafter referred to as *Sse*I) sites of a plasmid pTL7 (Japanese Patent Application No. 2003-92827), and a vector plasmid pTLGUSAmpcodN for use in the introduction of recognition sequences to a host DNA was prepared (Fig. 15C) by inserting two RSs facing opposite directions and the region inserted between them, which had been cut out from the above-described pCAmpCodN with the restriction enzyme *Sse*I*,* into the restriction enzyme *Ss*eI site of this pTLGUS. When this vector is introduced into a plant cell, the region between right border (RB) and left border (LB) of T-DNA shown by a small black triangle in the drawing is introduced into a plant DNA.

### II. Construction of the vector of the invention (p2nd1)

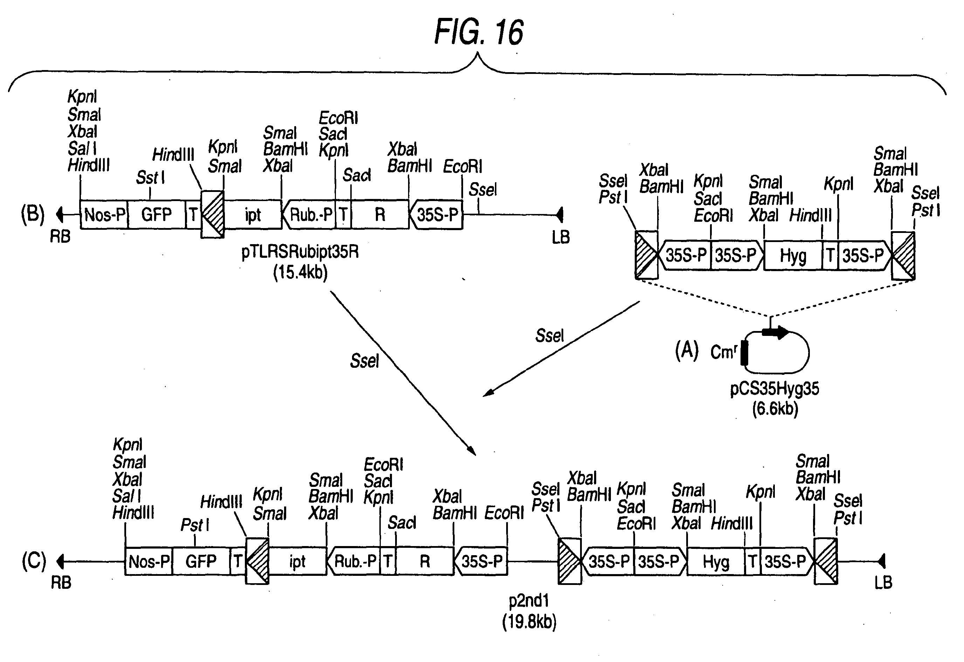

A plasmid pCS35hyg35 having the structure shown in Fig. 16A was constructed. In this case, the region inserted between the opposite direction RSs becomes an introduction cassette. Also, Hyg in the drawing represents a hygromycin resistant structural gene.

On the other hand, a plasmid pTLRSrubipt35R was constructed (Fig. 16B) by introducing a jellyfish fluorescent protein (GFP) structural gene to which Nos-P and polyadenylation signal were ligated, to a restriction enzyme *Hind*III site of pTL7 (H. Ebinuma et al., Molecular Methods of Plant Analysis, 22: 95 (2002)), and an *ipt* structural gene (contains a terminator sequence of the *ipt* gene itself) ligated to the Rubisco promoter (Rub-P), to the region between restriction enzymes *Eco*RI and *Sma*I sites, a site-specific recombinase (R) structural gene ligated to 35S-P and polyadenylation signal, to the *Eco*RI site, and further an RS sequence to the region between restriction enzymes *Hind*III and *Sma*I sites. Also, the R gene is a gene encoding a site-specific recombinase which catalyzes the site-specific recombination reaction by recognizing RS.

Subsequently, the introduction cassette moiety of pCS35hyg35 and the RS sequences inserting this were cut out as a whole with a restriction enzyme *Sse*I and ligated to the restriction enzyme *Sse*I site of pTLRSrubipt35R to prepare the vector plasmid p2nd1 of the invention (Fig. 16C), and this was applied to International Depositary (deposition number: FERM BP-8387, depositary authority: International Patent Organism Depositary, National Institute of Advanced Industrial Science and Technology (Central 6, 1-1, Higashi 1-chome, Tsukuba-shi, Ibaraki 305-8566, JAPAN), deposited on May 27, 2003).

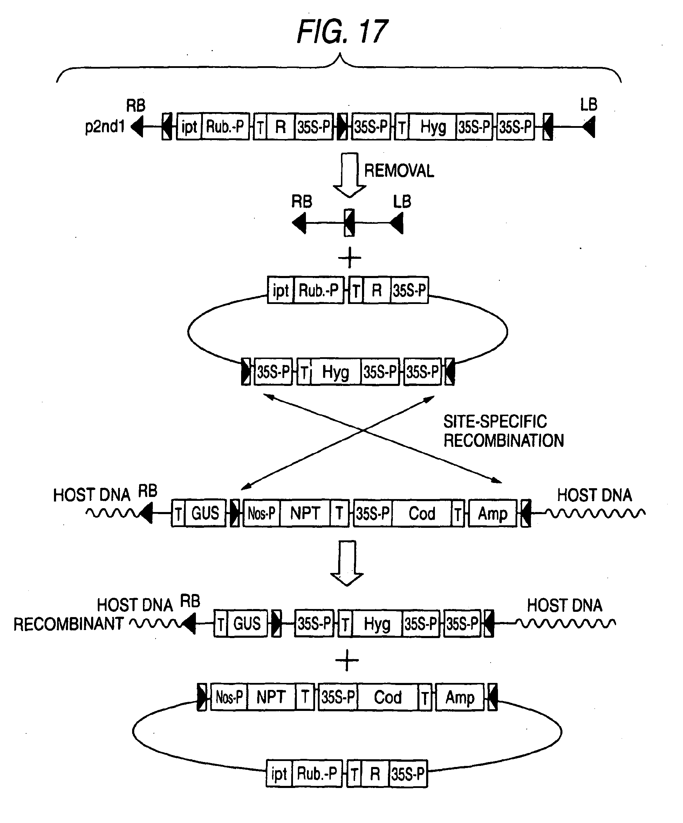

This vector is a removal type vector in which 35S-P is positioned as a model of the desired gene inside the introduction cassette, a hygromycin (Hyg) gene is positioned as the selectable marker gene therein, and a recombinase gene and a morphological abnormality induction gene are positioned outside it, and the GUS gene is expressed when site-specific recombination is caused between this and a host DNA into which a recognition sequence is introduced by the above-described pTLGUSAmpcodN, and 35S-P is introduced as the desired gene to the target position (Fig. 17). In addition, a part of the host DNA is deleted when this is introduced into the same DNA of the DNA into which the recognition sequence is introduced by a non-specific recombination.

### III. Introduction of vector into Agrobacterium

*A. tumefaciens* 4404 was inoculated into 10 ml of a YEB liquid medium (beef extract 5 g/l, yeast extract 1 g/l, peptone 1 g/l, sucrose 5 g/l, 2 mM MgSO₄, pH 7.2 at 22°C (unless otherwise indicated, hereinafter pH at 22°C)), and cultured at 28°C until OD630 reached a range of from 0.4 to 0.6. After collecting the cells by centrifuging the culture at 6900 × g and at 4°C for 10 minutes, the cells were suspended in 20 ml of 10 mM HEPES (pH 8.0), the cells were again collected by centrifuging at 6900 × g and at 4°C for 10 minutes, and the thus obtained cells were suspended in 200 µl of YEB liquid medium and used as the cell suspension for plasmid introduction.

Next, 50 µl of the cell suspension for plasmid introduction and 3 µl for each of the pTLGUSAmpcodN and p2nd1 prepared in I and II were mixed in a 0.5 ml-tube, and electroporation (Gene Pulser II system [BIO RAD] was used) was carried out to introduce these vectors into *A. tumefaciens* 4404. The cells after electroporation were cultured on a shaker at 25°C for 1 hour by adding 200 µl of the YEB liquid medium, and then inoculated onto an YEB agar medium (agar 1.5 w/v %, other compositions are as described above) supplemented with 50 mg/l kanamycin and cultured at 28°C for 2 days.

As a result of this culturing, each of the cells which formed bacterial colonies was further inoculated into the YEB liquid medium and cultured, and then, plasmid was extracted by the alkali method, and this was digested with *Eco*RI or *Hin*dIII and subjected to polyacrylamide gel electrophoresis to analyze lengths of the restriction enzyme digests, thereby confirming introduction of pTLGUSAmpcodN or p2nd1 into *A. tumefaciens* 4404. Also, the bacterium in which introduction of pTLGUSAmpcodN was confirmed was named LBA4404(pTLGUSAmpcodN), and the bacterium in which introduction of p2nd1 was confirmed was named LBA4404(p2nd1).

### IV. Introduction of vector for recognition sequence introduction (pTLGUSAmpcodN) into tobacco plant

The pTLGUSAmpcodN was introduced into a tobacco plant by infecting the LBA4404 (pTLGUSAmpcodN) obtained in III to the tobacco plant. As the material for pTLGUSAmpcodN introduction, a piece of leaf was used which was obtained by removing main vein from a leaf of a tobacco plant (*Nicotiana tabacum* SR1, hereinafter the same shall apply, unless otherwise indicated) grown in a culture vessel, and cutting the leaf into about 8 mm square.

That is, after soaking 36 leaf pieces obtained in the above-described manner in a cell suspension of LBA4404 (pTLGUSAmpcodN) (OD630 = 0.25, after culturing overnight using the YEB liquid medium, the cell density was adjusted by diluting with sterile water) for about 1 minutes, and they were put on a sterilized filter paper to remove excess cell suspension and then put on the MS agar medium (T. Murashige and F. Skoog, Physiol. Plant., 15: 473 (1962), to which 0.8 w/v % agar was added) which does not contain plant hormone (hormone-free) and is supplemented with 50 mg/l of acetosyringone, with the back of leaf upside, and cultured at 25°C under a total light (unless otherwise indicated, culturing of explants, plant tissues and plant bodies was carried out under these conditions) for 3 days to thereby infect these leaf pieces with LBA4404 (pTLGUSAmpcodN).

Adventitious buds were re-differentiated from the above-described leaf pieces after infection treatment, by transplanting and culturing them on MS agar medium supplemented with 1 mg/l of 6-benzylaminopurine, 0.1 mg/l of naphthaleneacetic acid, 500 mg/l of carbenicillin and 200 mg/l of kanamycin. The re-differentiated adventitious buds were separated, and transplanted and cultured on the hormone-free MS agar medium supplemented with 500 mg/l of carbenicillin and 200 mg/l of kanamycin for rooting. In this way, it was able to obtain 10 lines of pTLGUSAmpcodN-introduced kanamycin resistant recombinants respectively derived from 10 adventitious buds. In addition, regarding 6 lines among the thus obtained 10 lines of kanamycin resistant recombinants, seeds were collected from rooted individuals after acclimation and sowed on the hormone-free MS agar medium containing 200 mg/l of kanamycin, and seedlings germinated and grown showing kanamycin-resistance were selected in 2 strains per 1 line.

### V. Analysis of tobacco plant into which the vector for recognition sequence introduction (pTLGUSAmpcodN) was introduced

### A. Southern analysis

Chromosomal DNA was extracted using the CTAB method from each of 12 plant strains (No. 2-1, 2-2, 4-1, 4-2, 5-1, 5-2, 6-1, 6-2, 7-1, 7-2, 10-1, 10-2) of the 6 kanamycin-resistant recombinant lines obtained in IV, and this was digested with restriction enzyme *Hind*III and subjected to 0.8% agarose gel electrophoresis. After subjecting the agarose gel after electrophoresis to alkali and acid treatments, its electrophoresis face was introduced on a nylon membrane, and hybridization of the nylon membrane was carried out using a prove P1 having homology with Amp gene, which had been labeled in advance using DIG PCR labeling kit (purchased from Boehringer Mannheim). When chemiluminescence detection was carried out after the hybridization using DIG Wash and Block Buffer (purchased from Boehringer Mannheim), it was confirmed that one copy of pTLGUSAmpcodN was introduced into 1 line, 2 strains (No. 2-1, 2-2) subjected to the Southern analysis.

### B. IPCR analysis

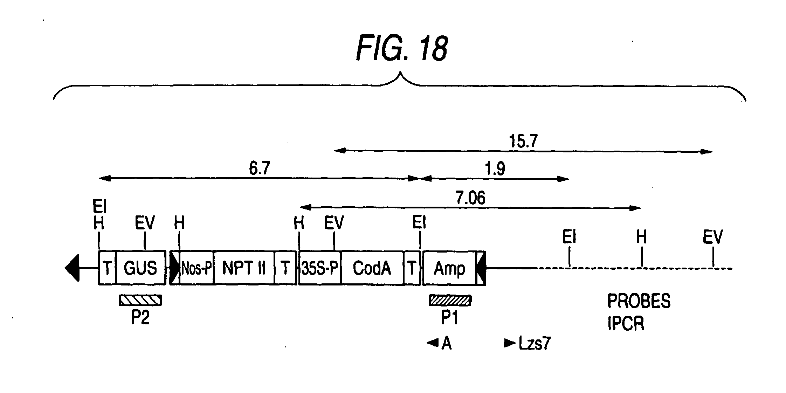

IPCR analysis of the strain 2-2 in which one copy of pTLGUSAmpcodN introduction was confirmed in the above-described A was carried out in the following manner using a primer A represented by SEQ ID NO:3 (5'-tgaacgaaat agacagatcg-3') having homology with Amp gene of pTLGUSAmpcodN and a primer Lzs7 represented by SEQ ID NO:4 (5'-attcgcaggg gataacgcag gaaa-3') having homology with the T-DNA region near the left border of pTLGUSAmpcodN (Fig. 18). As shown by the results of Southern analysis, when one copy of pTLGUSAmpcodN is introduced into this recombinant, a DNA of about 600 bp outside of the T-DNA left border originated from pTLGUSAmpcodN will be amplified by PCR.

Firstly, chromosomal DNA was extracted from this strain 2-2 using the CTAB method, and this DNA was digested with restriction enzyme *Eco*RI, subjected to ethanol precipitation treatment and then made into a cyclic form. Then, 1 µg of this cyclic DNA was dissolved in 50 µl of a PCR buffer solution prepared by mixing 10 mM Tris-HCl (pH 8.8 at 25°C) containing 0.2 µM of each of the primer A and primer Lzs7, 50 mM KCl, 1.5 mM MgCl₂, 1 w/v % Triton X-100, 0.1 mM dNTP and 1.25 units of Taq polymerase (purchased from CETUS), this mixed liquid was subjected to PCR by heating this at 94°C for 1 minute and 30 seconds and then repeating a heating cycle of 30 seconds at 94°C, 30 seconds at 60°C and 2 minutes at 72°C, 30 times, and the thus obtained PCR reaction product was analyzed by agarose gel electrophoresis.

As a result of the above-described agarose gel electrophoresis, amplification of a DNA fragment of about 600 bp was detected, and introduction of one copy of pTLGUSAmpcodN into the recombinant strain 2-2 was able to be confirmed also by the IPCR analysis. Also, when the thus amplified DNA fragment was subcloned using TOPO-DNA Cloning kit for sequencing (purchased from Invitrogen), and the DNA sequence was determined using a DNA sequencer, it was revealed that the T-DNA region of pTLGUSAmpcodN was introduced into the DNA of this recombinant, in such a state that its left border of about 200 bp was deleted.

### VI. Introduction of the vector of the invention (p2nd1) into recognition sequence-introduced tobacco plant

By infecting the recombinant strain 2-2 in which introduction of one copy of pTLGUSAmpcodN was confirmed in V with the LBA4404(p2nd1) obtained in III, the vector p2nd1 of the invention was introduced into this recognition sequence-introduced tobacco plant. The infection treatment was carried out in the same manner as in IV.

When the tobacco leaf pieces after infection treatment were transplanted into the MS agar medium containing 1 mg/ml of naphthaleneacetic acid, 0.1 mg/ml of benzyadenine, 500 mg/ml of carbenicillin and 20 mg/ml of hygromycin and the culturing was continued, 26 calli were obtained from 32 leaf pieces, and 60 adventitious buds were obtained by transplanting the 26 calli into the same composition of medium and continuing the culturing for 4 weeks. It was able to obtain 49 rooted individuals by continuing subculture of these adventitious buds using the hormone-free MS agar medium.

### VII. Analysis of tobacco plant into which recognition sequence and the vector of the invention (p2nd1) were introduced

### A. PCR analysis

Chromosomal DNA was extracted from each of the 49 rooted individuals obtained in VI using Fast DNA Kit (BIO 101 Inc.), and OCR analysis was carried out under the same conditions of V-B, using the primer H1 represented by SEQ ID NO:5 (5'-cgtctgtcga gaagtttctg-3') and the primer H2 represented by SEQ ID NO:6 (5'-ctatcggcga gtacttctac-3'), which bind to Hyg gene, the primer S represented by SEQ ID NO:7 (5'-acaatcccac tatccttcgc-3') which binds to 35S-O and the primer G represented by SEQ ID NO:8 (5'-tgcatcggcg aactgatcgt-3') which binds to GUS gene, and further, the primer GE represented by SEQ ID NO:9 (5'-cagcagggga aggggatag-3') which binds to the outside DNA of the T-DNA left border originate from pTLGUSAmpcodN, whose DNA sequence was determined by the IPCR method carried out in V-B, and the above-described S.

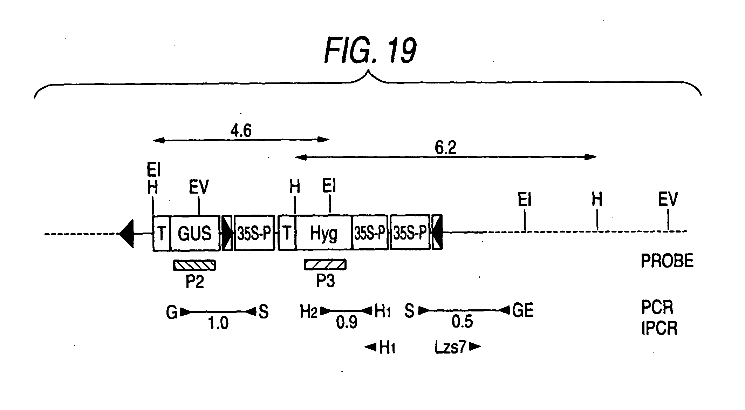

In this case, when the Hyg structural gene which was present inside the introduction cassette of p2nd1 was introduced into the analyzed DNA, a DNA fragment of about 900 bp (H1-H2) is amplified. Also, when the region inserted between the recognition sequences on the tobacco plant chromosomal DNA, introduced by pTLGUSAmpCodN, was substituted with the introduction cassette of p2nd1 as a result of site-specific recombination, DNA fragments of about 1 kbp (S-G) and about 500 bp (GE-S) are amplified. In addition, when amplification of only one of the about 1 kbp (S-G) and about 500 bp(GE-S) was detected, it is considered that a part of DNA of the individual is deleted or inverted (Fig. 19).

As a result of the PCR analysis, amplification of a DNA fragment of about 900 bp was detected in all of the 49 analyzed individuals. In addition, amplification of 1 kbp was detected in 10 individuals, amplification of 500 bp was detected in 12 individuals, and amplification of 1 kbp and 500 bp were detected in 5 individuals (2-2-14, 2-2-20, 2-2-27, 2-2-28, 2-2-29), and these 5 individuals in which amplification of 1 kbp and 500 bp were detected were selected as recombinants in which the desired gene was introduced into the predetermined position on the DNA.

### B. Southern analysis

Southern analysis of the recombinants (2-2-14, 2-2-20, 2-2-27, 2-2-28, 2-2-29) selected in A was carried out in the same manner as in V-A. In this case, however, the chromosomal DNA was digested with a restriction enzyme *Eco*RI or *Hind*III, and a probe P2 having homology with GUS gene (when chromosomal DNA was digested with *Eco*RI) or a probe P3 having homology with Hyg gene (when chromosomal DNA was digested with *Hind*III) was used as the probe.

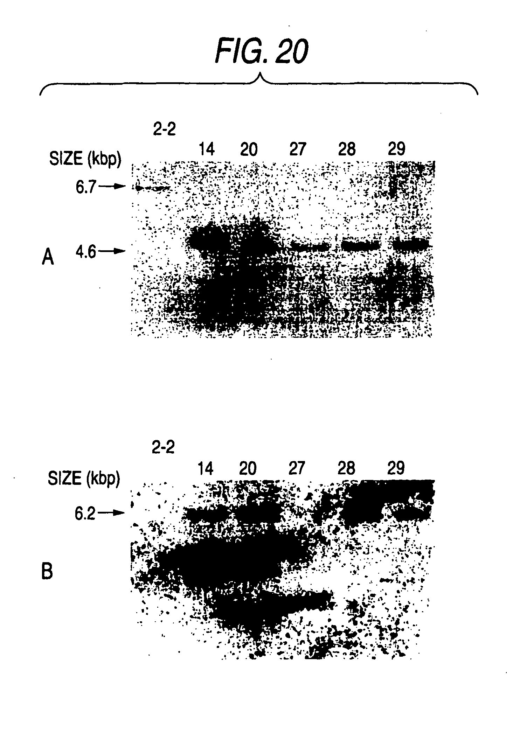

Fig. 20A shows a result when the chromosomal DNA of each of the above-described recombinants was digested with *Eco*RI, and the probe P2 having homology with GUS gene was used as the probe. A band of 4.6 kbp was confirmed in all of the strains 2-2-14, 2-2-20, 2-2-27, 2-2-28 and 2-2-29. This coincides with the length of an *Eco*RI fragment which was predictable when the region inserted between the recognition sequences on the respective chromosomal DNA, which were introduced by pTLGUSAmpCodN, was substituted with the introduction cassette of p2nd1 as a result of site-specific recombination (Fig. 19). On the other hand, a band of 6.7 kbp was confirmed in the recombinant strain 2-2 of before the p2nd1 introduction, and this also coincides with the length of an *Eco*RI fragment which was predictable when only the construction by pTLGUSAmpCodN was introduced on the chromosomal DNA (Fig. 18).

Also, Fig. 20B shows a result when chromosomal DNA of each of them was digested with *Hind*III, and the probe P3 having homology with Hyg gene was used as the probe. A band of 6.2 kbp was confirmed in all of the strains 2-2-14, 2-2-20, 2-2-27, 2-2-28 and 2-2-29. This also coincides with the length of an *Hind*III fragment which was predictable when the region inserted between the recognition sequences on the respective chromosomal DNA, which were introduced by pTLGUSAmpCodN, was substituted with the introduction cassette of p2nd1 (Fig. 19). In addition, as a result of the Southern analysis, it was confirmed that one copy of the Hyg gene is introduced into 4 individuals of other than the strain 2-2-27.

### C. IPCR analysis

IPCR analysis of the recombinants selected in A (2-2-14, 2-2-20, 2-2-27, 2-2-28, 2-2-29) was carried out in the same manner as in V-B, using the primer Lzs 7 and the primer H1 having homology with the Hyg gene (Fig. 19). As the results of Southern analysis carried out in the above-described B show, when substitution is caused between the region inserted between the recognition sequences and the introduction cassette of p2nd1, among the constructions introduced by pTLGUSAmpCodN, in the DNA of each of these recombinants, there is no difference between these recombinants and the recombinant into which the construction by pTLGUSAmpcodN alone was introduced, in terms of the DNA sequence of the T-DNA left border region originated from pTLGUSAmpcodN and the outside thereof, so that the DNA of about 600 bp should be amplified in this case, too.

As a result of the IPCR analysis, the chromosomal DNA of each of the recombinants selected in A (2-2-14, 2-2-20, 2-2-27, 2-2-28, 2-2-29) showed amplification of the DNA fragment of about 600 bp, and when DNA sequences of these DNA fragments were analyzed in the same manner as in V-B, the DNA sequence of the T-DNA left border region and the outside thereof coincided with the DNA sequence of the recombinant into which the construction by pTLGUSAmpcodN alone was introduced. Accordingly, it is considered that the results of this IPCR analysis support the results of the Southern analysis.

As a result of the above PCR analysis, Southern analysis and IPCR analysis, it was confirmed that the NPTII gene, codA gene and Amp gene between the RS sequences mutually facing opposite directions, introduced on the DNA by pTLGUSAmpCodN, are substituted with the introduction cassette of p2nd1, in the strain 2-2-14, strain 2-2-20, strain 2-2-27, strain 2-2-28 and strain 2-2-29. That is, according to the invention, it was able to obtain 5 recombinant individuals in which a gene was introduced into a predetermined position of chromosomal DNA, using 32 sections as the materials, and its efficiency was 1.6×10⁻¹. This is 100 times or higher efficiency than the result obtained by the above-described transient method (Vergunst *et al*. 1998) and is also 10 times or higher efficiency when compared with the result obtained by a method in which expression of a recombinase is shut off by the introduction of a desired gene to a target position (Vergunst *et al.* 1998).

In addition, based on the above results, it was also revealed that deletion or inversion of a host DNA can be attained efficiently by this vector p2nd1, without a crossing step. That is, based on the results of PCR analysis carried out in the above-described A, it is considered that the individual in which amplification of a DNA fragment of 1 kbp alone was detected and the individual in which amplification of a DNA fragment of 500 bp alone was detected are the recombinants in which such a deletion or inversion of DNA occurred.

### Example 2

### 1. Construction of the vector of the invention (p2ndΔRSipt)

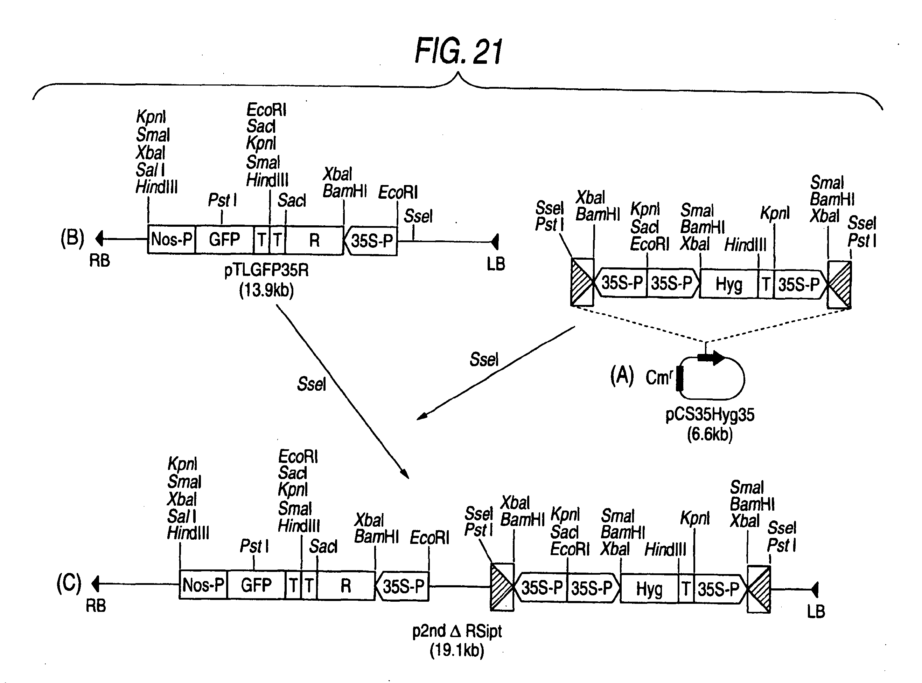

A plasmid pTLGFP35R was constructed by introducing a GFP structural gene, to which Nos-P and polyadenylation signal were ligated, into the restriction enzyme *Hind*III site of pTL7 (Japanese Patent Application No. 2003-92827), and an R structural gene, to which 35S-P and polyadenylation signal were ligated, into the *Eco*RI site (Fig. 21B).

Subsequently, the introduction cassette region of the pCS35hyg35 constructed in II of Example 1 was cut out, together with the RS sequences inserting this, by restriction enzyme *Sse*I and ligated to the restriction enzyme *Sse*I site of pTLGFP35R to prepare the vector plasmid p2ndΔRSipt of the invention, and this was internationally deposited (deposition number: FERM BP-08560, depositary authority: International Patent Organism Depositary, National Institute of Advanced Industrial Science and Technology (Central 6, 1-1, Higashi 1-chome, Tsukuba-shi, Ibaraki 305-8566, JAPAN), deposited on December 3, 2003).

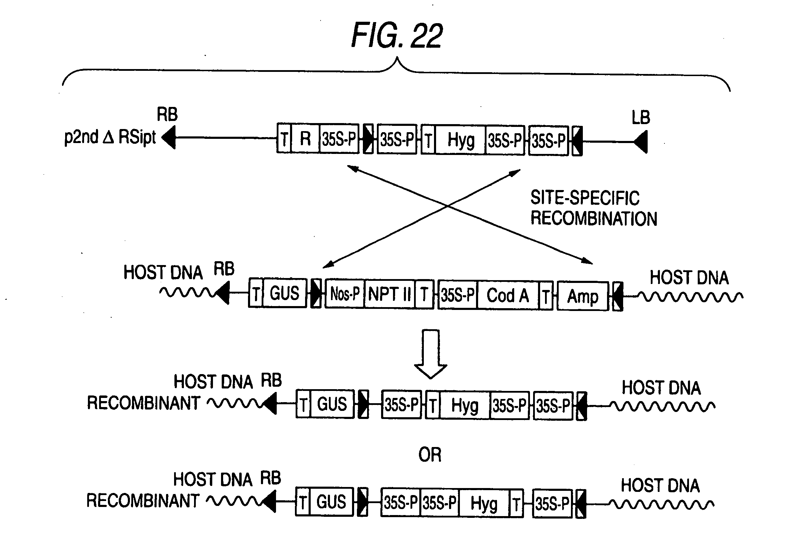

This vector is a vector in which 35S-p is positioned as a model of the desired gene, and the Hyg gene is positioned as the selectable marker gene, inside the introduction cassette, and a recombinase gene is positioned outside the introduction cassette, so that the GUS gene is expressed when 35S-P as the desired gene is introduced into a target position due to site-specific recombination occurred between this and the host DNA into which a recognition sequence was introduced by the above-described pTLGUSAmpcodN (Fig. 22).

### II. Introduction of the vector of the invention (p2ndΔRSipt) into Agrobacterium

In the same manner as in III of Example 1, the p2ndΔRSipt prepared in the above-described I was introduced into *A. tumefaciens* 4404, this was confirmed by the DNA analysis, and this strain was named LBA4404 (p2ndΔRSipt).

### III. Introduction of the vector of the invention (p2ndΔRSipt) into recognition sequence-introduced tobacco plant

By infecting the LBA4404(p2ndΔRSipt) obtained in II to the recombinant strain 2-2 in which introduction of one copy of pTLGUSAmpcodN was confirmed in V of Example 1, the vector p2ndΔRSipt of the invention was introduced into this recognition sequence-introduced tobacco plant. The infection treatment was carried out in the same manner as in IV of Example 1.

When the tobacco leaf pieces after the infection treatment were transplanted into the MS agar medium containing 1 mg/ml of naphthaleneacetic acid, 0.1 mg/ml of benzyadenine, 500 mg/ml of carbenicillin and 20 mg/ml of hygromycin and the culturing was continued, 63 calli were obtained from 32 leaf pieces, and 239 adventitious buds were obtained by transplanting the 63 calli into the same composition of medium and continuing the culturing for 4 weeks. A total of 53 rooted individuals were obtained by continuing subculture of these adventitious buds using the hormone-free MS agar medium.

### IV. Analysis of tobacco plant into which recognition sequences and the vector of the invention (p2ndΔRSipt) were introduced

### A. PCR analysis

PCR analysis of the 53 rooted individuals obtained in III was carried out using the same primers and conditions of VII-A in Example 1.

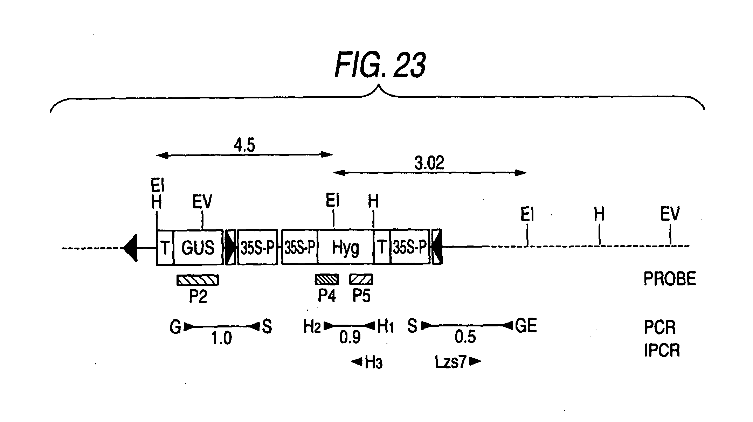

In this case too, when the Hyg structural gene which was present inside the introduction cassette of p2ndΔRSipt was introduced into the analyzed DNA, a DNA fragment of about 900 bp (H1-H2) is amplified. Also, when the region inserted between the recognition sequences on the tobacco plant chromosomal DNA, introduced by pTLGUSAmpCodN, was substituted with the introduction cassette of p2ndΔRSipt, DNA fragments of about 1 kbp (S-G) and about 500 bp(GE-S) are amplified. In addition, when amplification of only one of the about 1 kbp (S-G) and about 500 bp(GE-S) was detected, it is considered that a part of DNA of the individual is deleted or inverted (Fig. 23).

As a result of the PCR analysis, amplification of a DNA fragment of about 900 bp was detected in all of the 53 individuals subjected to the analysis. In addition, amplification of 1 kbp was detected in 3 individuals, amplification of 500 bp was detected in 2 individuals, and amplification of 1 kbp and 500 bp were detected in 2 individuals (30-1, 91-1), and these 2 individuals in which amplification of 1 kbp and 500 bp were detected were selected as recombinants in which the desired gene was introduced into the predetermined position on the DNA.

### B. Southern analysis

Southern analysis of the recombinants (30-1, 91-1) selected in A was carried out in the same manner as in V-A of Example 1. In this case, however, the chromosomal DNA was digested with a restriction enzyme *Eco*RI, and a probe P4 having homology with Hyg gene or a probe P5 also having homology with Hyg gene was used as the probe.

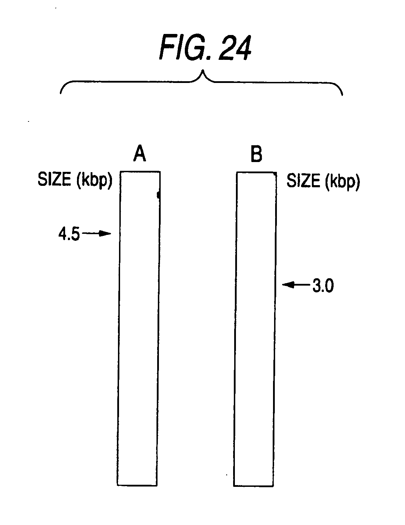

Fig. 24A shows a result when the chromosomal DNA of the recombinant 91-1 was digested with *Eco*RI*,* and the probe P4 was used as the probe. As is apparent from Fig. 24A, a band of 4.5 kbp was confirmed in this case as a result of the Southern analysis. In addition, Fig. 24B also shows a result when the chromosomal DNA of the recombinant 91-1 was digested with *Eco*RI, and the probe P5 was used as the probe. As is apparent from Fig. 24B, a band of 3.0 kbp was confirmed in this case. These bands coincide with the lengths of *Eco*RI fragments which are predictable when the regions inserted between the respective recognition sequences on these chromosomal DNA molecules, which were introduced by pTLGUSAmpCodN, were substituted with the introduction cassette of p2ndΔRSipt as a result of site-specific recombination (Fig. 23). Also, as a result of this Southern analysis, it was confirmed that one copy of the Hyg gene is introduced into the strain 91-1. In addition, results similar to those of the strain 90-1 were able to be obtained also from the recombinant strain 30-1.

### C. IPCR analysis

IPCR analysis of the recombinant selected in A (91-1) was carried out in the same manner as in V-B of Example 1, using the primer Lzs7 and the primer H3 represented by SEQ ID NO:10 (5'-gatgcaatag gtcagctct-3') having homology with the Hyg gene. Similar to the case of VII-C of Example 1, as the results of Southern analysis carried out in the above-described B show, when substitution is caused between the region inserted between the recognition sequences and the introduction cassette of p2ndΔRSipt, among the constructions introduced by pTLGUSAmpCodN, in the DNA of each of these recombinants, there is no difference between these recombinants and the recombinant into which the construction by pTLGUSAmpCodN alone was introduced, in terms of the DNA sequence of the T-DNA left border region originated from pTLGUSAmpcodN and the outside thereof, so that the DNA of about 600 bp should be amplified by PCR in this case, too.

As a result of the IPCR analysis, the chromosomal DNA of the recombinant strain 91-1 showed amplification of the DNA fragment of about 600 bp, and when DNA sequences of these DNA fragments were analyzed in the same manner as in V-B of Example 1, it was revealed that the DNA sequence of the T-DNA left border region and the outside thereof coincides with the DNA sequence of the recombinant into which the construction by pTLGUSAmpCodN alone was introduced. Accordingly, it is considered that the results of this IPCR analysis support the results of the Southern analysis.

As a result of the above PCR analysis, Southern analysis and IPCR analysis, it was confirmed that the NPTII gene, codA gene and Amp gene between the RS sequences mutually facing opposite directions, introduced on the DNA by pTLGUSAmpCodN, are substituted with the introduction cassette of p2ndΔRSipt, in the recombinant strain 30-1 and strain 91-1. That is, according to the invention, it was able to obtain 2 recombinant individuals in which a gene was introduced into a predetermined position of the chromosomal DNA, using 32 sections as the materials, and its efficiency was 6.3×10⁻². This efficiency is also 100 times or higher efficiency than the result obtained by the above-described transient method (Vergunst *et al*. 1998).

In addition, it was also revealed that deletion or inversion of a host DNA at a predetermined position can be attained efficiently by this vector p2ndΔRSipt, without a crossing step. That is, based on the results of PCR analysis carried out in the above-described A, it is considered that the individual in which amplification of a DNA fragment of 1 kbp alone was detected is the recombinant in which such a deletion or inversion of DNA is caused.

### Example 3

### 1. Construction of the vector of the invention (p2nd20)

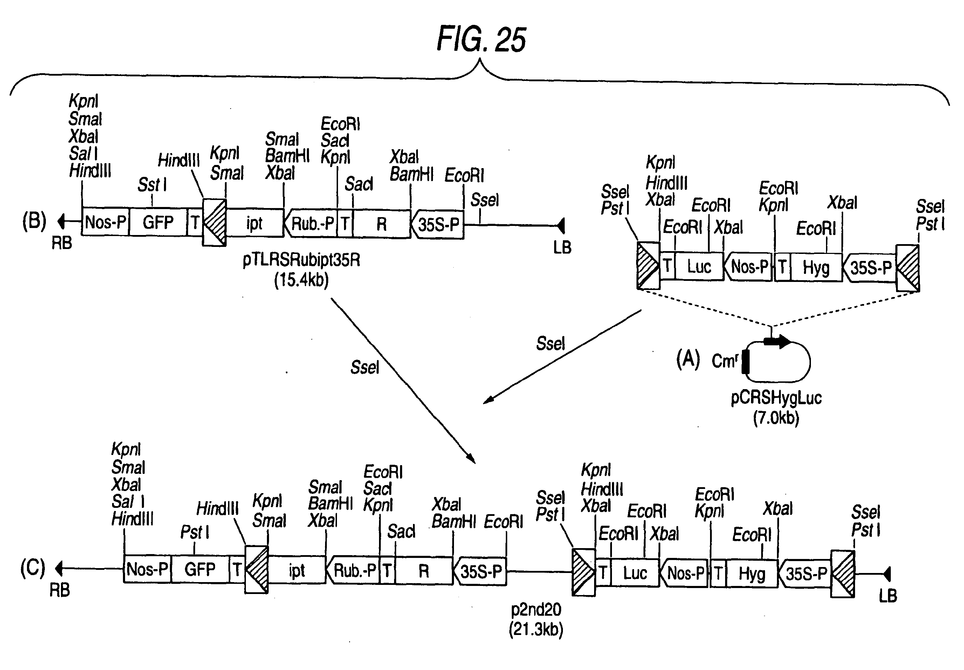

A plasmid pCRSHygLuc having the structure shown in Fig. 25A was constructed. Like the case of Example 1, the region inserted between the RS of opposite directions becomes the introduction cassette in this case, too. Also, the Luc in the drawing represents a luciferase structural gene.

Subsequently, the introduction cassette region of this pCRSHygLuc was cut out, together with the RS sequences inserting this, by restriction enzyme *Sse*I and ligated to the restriction enzyme *Sse*I site of the plasmid pTLRSrubipt35R constructed in II of Example 1 (Fig. 25B) to prepare the vector plasmid p2nd20 of the invention (Fig. 25C), and this was internationally deposited (deposition number: FERM BP-08561, depositary authority: International Patent Organism Depositary, National Institute of Advanced Industrial Science and Technology (Central 6, 1-1, Higashi 1-chome, Tsukuba-shi, Ibaraki 305-8566, JAPAN), deposited on December 3, 2003).

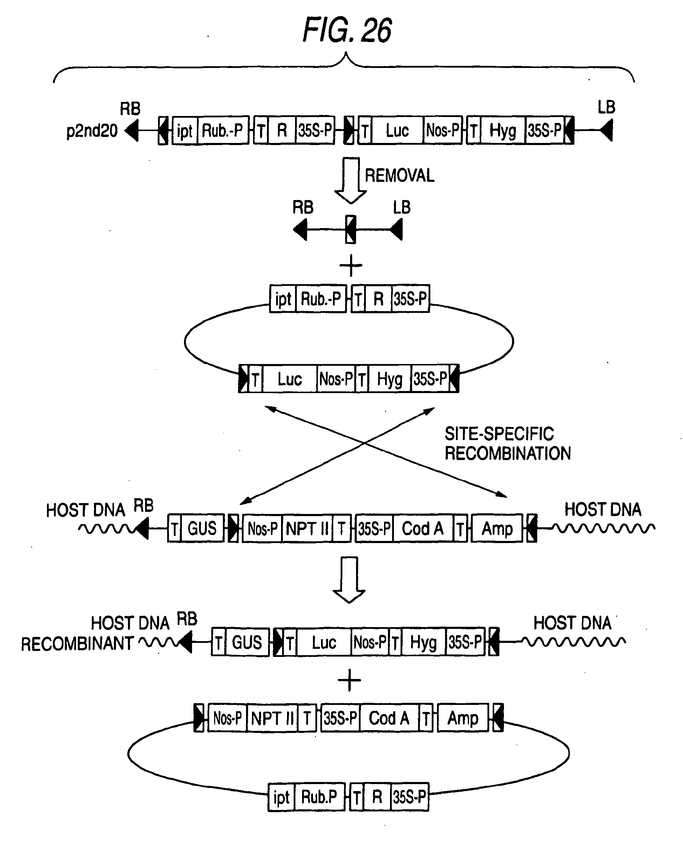

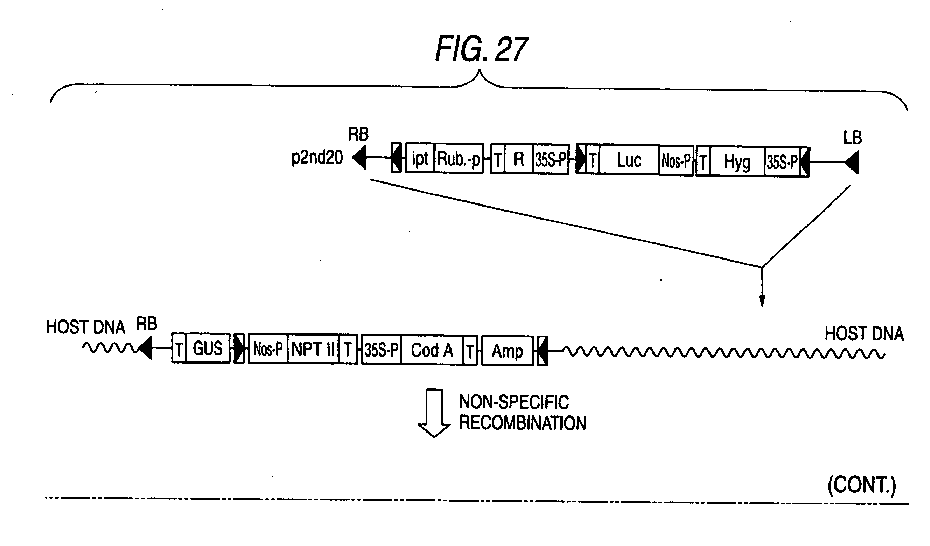

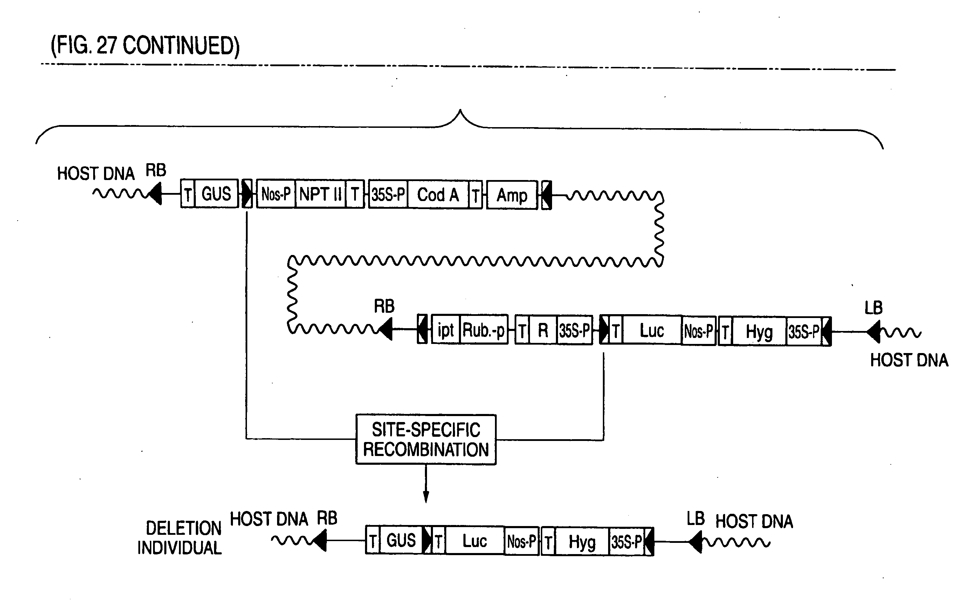

This vector is a removal type vector in which the luciferase (Luc) gene is positioned as a model of the desired gene, and the Hyg gene is positioned as the selectable marker gene, inside the introduction cassette, and a recombinase gene and a morphological abnormality induction gene are positioned outside it, so that the Luc gene as the desired gene is introduced into the target position when site-specific recombination is caused between this and the host DNA into which a recognition sequence was introduced by the above-described pTLGUSAmpcodN (Fig. 26). In addition, this is constructed such that when introduced into a chromosome identical to the chromosome into which recognition sequences are introduced by a non-specific recombination, introduction of the desired gene to the target position and deletion of the host DNA at the predetermined position simultaneously are caused by the mechanism shown in Fig. 27.

### II. Introduction of the vector of the invention (p2nd20) into Agrobacterium

In the same manner as in III of Example 1, the p2nd20 prepared in the above-described I was introduced into *A. tumefaciens* 4404, this was confirmed by the DNA analysis, and this strain was named LBA4404(p2nd20).

### III. Introduction of the vector of the invention (p2nd20) into recognition sequence-introduced tobacco plant

By infecting the LBA4404(p2nd20) obtained in II to the recombinant strain 2-1 in which introduction of one copy of pTLGUSAmpcodN was confirmed in V of Example 1, the vector p2nd20 of the invention was introduced into this recognition sequence-introduced tobacco plant. The infection treatment was carried out in the same manner as in IV of Example 1.

When the tobacco leaf pieces after infection treatment were transplanted into the MS agar medium containing 1 mg/ml of naphthaleneacetic acid, 0.1 mg/ml of benzyadenine, 500 mg/ml of carbenicillin and 20 mg/ml of hygromycin and the culturing was continued, 60 calli were obtained from 64 leaf pieces, and 64 adventitious buds were obtained from 34 calli by transplanting the 60 calli into the same composition of medium and continuing the culturing for 4 weeks. A total of 31 rooted individuals were obtained from 21 lines of adventitious buds respectively originated from 21 calli, by continuing subculture of these adventitious buds using the hormone-free MS agar medium.

### IV. Analysis of tobacco plant into which recognition sequences and the vector of the invention (p2nd20) were introduced

### A. PCR analysis

A chromosomal DNA was extracted from each of the 31 rooted individuals obtained in III using Fast DNA Kit (BIO 101 Inc.), and OCR analysis was carried out under the same conditions of V-B, using the primers H1 and H2 which bind to Hyg gene, the primer L3 represented by SEQ ID NO:11 (5'-cgttcggttg gcagaagcta tgaaa-3') which binds to Luc structural gene and the primer G which binds to GUS gene, the primer HR1 represented by SEQ ID NO: 12 (5'-cagaaacttc tcgacagacg-3') which binds to Hyg gene and the above-described G, and further, the primer GE which binds to the outside DNA of the T-DNA left border originate from pTLGUSAmpcodN and the above-described L3 or the above-described HR1.

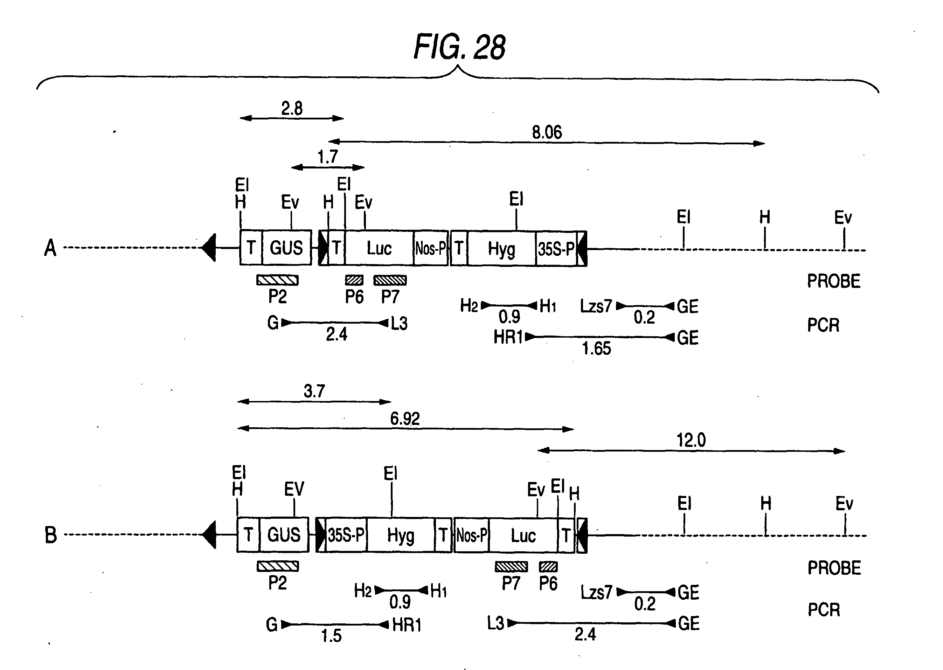

In this case, when the Hyg structural gene which was present inside the introduction cassette of p2nd20 was inserted into the analyzed DNA, a DNA fragment of about 900 bp (H1-H2) is amplified. Also, when the region inserted between the recognition sequences on the tobacco plant chromosomal DNA, introduced by pTLGUSAmpCodN, was substituted with p2nd20 due to site-specific recombination as shown in Fig. 28A, DNA fragments of about 2.4 kbp (G-L3) and about 1.65 kbp (GE-HR1) are amplified. On the other hand, when site-specific recombination is caused as shown in Fig. 28B, DNA fragments of about 1.5 kbp (G-HR1) and about 2.4 kbp (GEL3) are amplified.

As a result of the PCR analysis, amplification of a DNA fragment of about 900 bp was detected in all of the 31 individuals subjected to the analysis. Among these, amplification of DNA fragments of about 2.4 kbp (G-L3) and about 1.65 kbp (GE-HR1) was detected in 2 individuals (12-2, 37-1), and amplification of DNA fragments of about 1.5 kbp (G-HR1) and about 2.4 kbp (GE-L3) was detected in 4 individuals (12-1, 22-1, 22-2, 43-1), and these were selected as recombinants in which the desired gene was introduced into the predetermined position on the DNA.

In addition, as a result of the PCR analysis, there were 4 individuals (7-1, 11-1, 17-3, 40-1) including those in which only the amplification of DNA fragment of about 2.4 kbp (G-L3) or about 1.5 kbp (G-HR1) was detected. Accordingly, when the PCR analysis was carried out on them together with the recombinants selected in the foregoing in which the desired gene was introduced into the predetermined position, using the primer Lzs7 having homology with the region near the pTLGUSAmpcodN-originated T-DNA left border and the above-described GE, a fragment of about 200 bp was not amplified only in these individuals, thus revealing that all of these individual strain 7-1, strain 11-1, strain 17-3 and strain 40-1 are individuals in which a DNA of a certain region was deleted (hereinafter simply referred to as gene deletion individual in some cases) (Fig. 28A and B).

### B. Southern analysis

Southern analysis of the recombinants in which the desired gene was introduced into the predetermined position (12-1, 12-2, 22-1, 22-2, 37-1, 43-1) selected in A and the gene deletion individuals (7-1, 11-1, 17-3, 40-1) was carried out in the same manner as in V-A of Example 1. In this case, however, the chromosomal DNA was digested with a restriction enzyme *Eco*RI*, Hind*III or *Eco*RV, and a probe P2 having homology with GUS gene (when chromosomal DNA was digested with *Eco*RI), a probe P7 having homology with Luc gene (when chromosomal DNA was digested with *Hind*III) or a probe P6 also having homology with Luc gene (when chromosomal DNA was digested with *Eco*RV*)* was used as the probe.

As a result of the Southern analysis, it was confirmed that the NPTII gene, codA gene and Amp gene, which were considered to be introduced by pTLGUSAmpCodN on the DNA and present thereon, were substituted with the introduction cassette of p2nd20 in all of the recombinants in which the desired gene was introduced into the predetermined position (12-1, 12-2, 22-1, 22-2, 37-1, 43-1). In addition, at the same time, the results of this Southern analysis also show that the introduction cassette of p2nd20 is introduced onto the DNA of each recombinant in the two directions shown in Fig. 28A and B, thus supporting results of the PCR analysis.

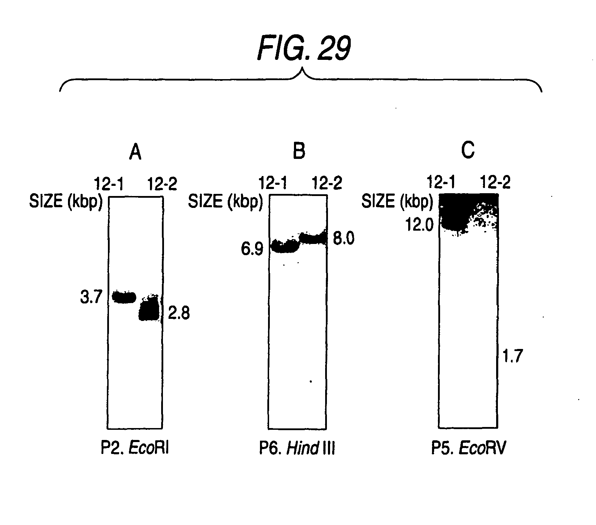

As an example, results of the Southern analysis of the recombinant strain 12-1 and strain 12-2 are shown. Fig. 29A shows a result when chromosomal DNA was digested with *Eco*RI, and the probe P2 was used as the probe, and in this case, a band of 3.7 kbp was detected in the recombinant strain 12-1, and a band of 2.8 kbp in the recombinant strain 12-2. Similar to the results of PCR analysis, this shows that the introduction cassette of p2nd20 was introduced at the direction shown in Fig. 28B in the case of the recombinant strain 12-1, and at the direction shown in Fig. 28A in the case of the recombinant strain 12-2.

Fig. 29B shows a result when the chromosomal DNA was digested with *Hind*III*,* and the probe P7 was used as the probe, and in this case, a band of 6.9 kbp was detected in the recombinant strain 12-1, and a band of 8.0 kbp in the recombinant strain 12-2. Similar to the results of PCR analysis, this also shows that the introduction cassette of p2nd20 was introduced at the direction shown in Fig. 28B in the case of the recombinant strain 12-1, and at the direction shown in Fig. 28A in the case of the recombinant strain 12-2.

Fig. 29C shows a result when chromosomal DNA was digested with *Eco*RV*,* and the probe P6 was used as the probe, and in this case, a band of 12.0 kbp was detected in the recombinant strain 12-1, and a band of 1.7 kbp in the recombinant strain 12-2. Similar to the results of PCR analysis, this also shows that the introduction cassette of p2nd20 was introduced at the direction shown in Fig. 28B in the case of the recombinant strain 12-1, and at the direction shown in Fig. 28A in the case of the recombinant strain 12-2.

Also, as a result of this Southern analysis, it was confirmed that one copy of the Luc gene was introduced into the target position in the case of the recombinants strain 12-1 and strain 12-2.

On the other hand, results of the Southern analysis show that a DNA of a certain region, which was present in the above-described strain 2-1, was deleted in all of the gene deletion individuals (7-1, 11-1, 17-3, 40-1), thus supporting the results of PCR analysis.

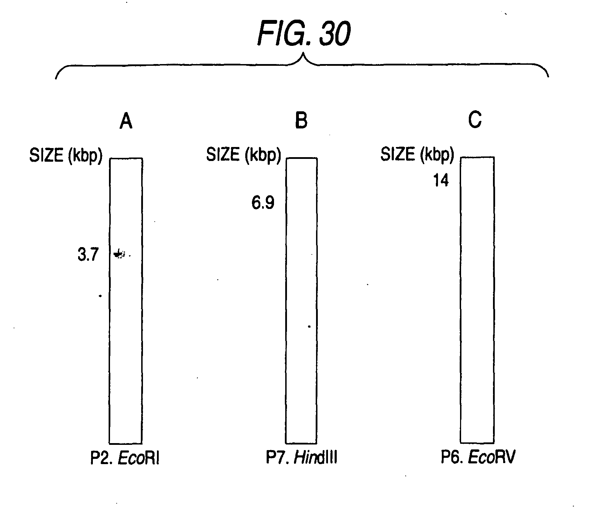

As an example, results of the analysis of the gene deletion individual strain 11-1 are shown in Fig. 30. As is apparent from Fig. 30, as a result of the Southern analysis, this gene deletion individual strain 11-1 showed a band of 3.7 kbp when the chromosomal DNA was digested with *Eco*RI and probe P2 was used as the probe, a band of 6.9 kbp when the chromosomal DNA was digested with *Hind*III and probe P7 was used as the probe, and a band of 14.0 kbp when the chromosomal DNA was digested with *Eco*RV and probe P6 was used as the probe.

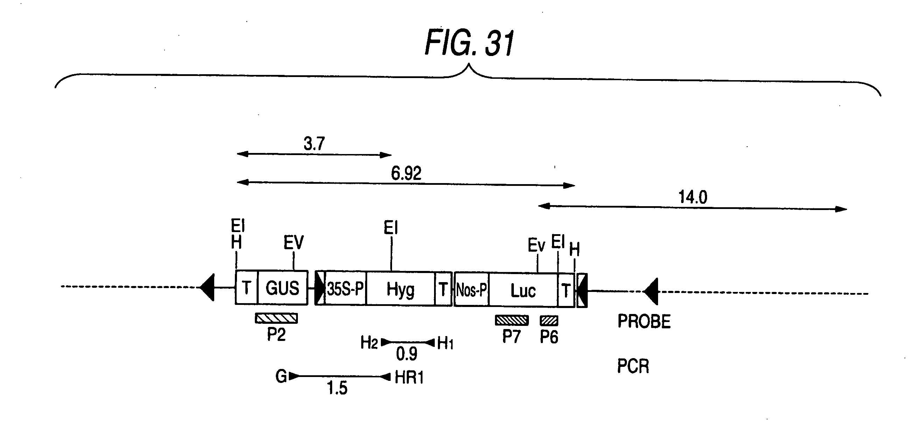

Each of these shows that, in this gene deletion individual strain 11-1, the introduction cassette of p2nd20 was introduced into the target position by the mechanism shown in Fig. 27, and at the same time, a part of the chromosomal DNA of the outside of the T-DNA left border originated from pTLGUSAmpcodN was deleted (Fig. 31).

As a result of the above PCR analysis and Southern analysis, it was confirmed that the NPTII gene, codA gene and Amp gene between the RS sequences mutually facing opposite directions, introduced on the DNA by pTLGUSAmpCodN, are substituted with the introduction cassette of p2nd20, in the strain 12-1, strain 12-2, strain 22-1, strain 22-2, strain 37-1 and strain 43-1, and these are recombinants in which the desired gene was introduced into the predetermined position of the chromosomal DNA. In addition, it was confirmed that the strain 7-1, strain 11-1, strain 17-3 and strain 40-1 are individuals in which a certain region of chromosomal DNA was deleted.

That is, according to the invention, it was able to obtain 6 recombinant individuals in which a gene was introduced into a predetermined position of the chromosomal DNA, using 64 sections as the materials, and its efficiency was 9.4×10⁻². This efficiency is also 100 times or higher efficiency than the result by the above-described transient method (Vergunst *et al*. 1998). In addition, it was able to obtain 4 individuals of the same generation in which a certain region of chromosomal DNA was deleted, without a crossing step, and its efficiency was 6.3×10⁻².

While the present invention has been described in detail and with reference to specific embodiments thereof, it will be apparent to one of skill in the art that various changes and modifications can be made therein without departing from the spirit and scope thereof

This application is based on Japanese patent application No. 2003-193227 filed on June 3, 2003 and Japanese patent application No. 2003-436431 filed on December 9, 2003.

### Industrial Applicability

According to the invention, one copy of a desired gene can be introduced into a predetermined position of a host DNA, at a high frequency without any bad influence upon host cells. In addition, the host DNA can also be deleted or inverted at a predetermined position, at a high frequency without a crossing step and without any bad influence upon host cells.

Thus, according to the invention, a recombinant which shows expression pattern of the desired gene and has high and stable expression quantity can be obtained with high efficiency.

## Claims

1. A vector comprising a first genetic moiety inserted between two site-specific recombinase recognition sequences, a site-specific recombinase gene which recognizes the site-specific recombinase recognition sequences, and a lethal induction gene or a morphological abnormality induction gene, wherein the site-specific recombinase gene and the lethal induction gene or the morphological abnormality induction gene are positioned outside the first genetic moiety.

2. The vector according to claim 1, wherein the first-genetic moiety, the site-specific recombinase gene and the lethal induction gene or the morphological abnormality induction gene are positioned such that they can be removed from the vector by recombinase expressed from the site-specific recombinase gene.

3. The vector according to claim 1, wherein the morphological abnormality induction gene is a plant hormone-related gene.

4. The vector according to claim 3, wherein the plant hormone-related gene is a cytokinin synthesis gene.

5. The vector according to claim 1, wherein a desired gene is positioned inside the first genetic moiety.

6. The vector according to claim 1, wherein a part or the whole of a selectable marker gene is positioned inside the first genetic moiety.

7. The vector according to claim 6, wherein at least a promoter moiety of the selectable marker gene is positioned inside the first genetic moiety.

8. The vector according to claim 6, wherein at least a structural gene moiety of the selectable marker gene is positioned inside the first genetic moiety.

9. A method for introducing a gene, which comprises introducing a vector comprising a first genetic moiety inserted between two site-specific recombinase recognition sequences, and a site-specific recombinase gene which recognizes the site-specific recombinase recognition sequences, and a lethal induction gene or a morphological abnormality induction gene which are positioned outside the first genetic moiety, into a host cell other than a human germ cell having a DNA in which one site-specific recombinase recognition sequence is present; and introducing the first genetic moiety into the host DNA by a site-specific recombination at the one site-specific recombinase recognition sequence on the host DNA.

10. The method for introducing a gene into a plant according to claim 9, wherein the host is a plant.

11. A method for introducing a gene, which comprises introducing a vector comprising a first genetic moiety inserted between two site-specific recombinase recognition sequences, and a site-specific recombinase gene which recognizes the site-specific recombinase recognition sequences, and a lethal induction gene or a morphological abnormality induction gene which are positioned outside the first genetic moiety, into a host cell other than a human germ cell having a DNA in which a moiety inserted between two site-specific recombinase recognition sequences is present; and introducing the first genetic moiety into the host DNA by a site-specific recombination at the two site-specific recombinase recognition sequences on the host DNA.

12. The method for introducing a gene into a plant according to claim 11, wherein the host is a plant.

13. A method for producing a plant tissue or a plant individual into which a desired gene is introduced, which comprising the following steps (A) to (C):
(A) introducing a vector comprising a first genetic moiety inserted between two site-specific recombinase recognition sequences, and comprising a desired gene inside the first genetic moiety, and a site-specific recombinase gene which recognizes the site-specific recombinase recognition sequences, and a lethal induction gene or a morphological abnormality induction gene which are positioned outside the first genetic moiety, into a plant cell having a DNA in which one site-specific recombinase recognition sequence is present or a DNA in which a moiety inserted between two site-specific recombinase recognition sequences is present,
(B) culturing the plant cell into which the vector is introduced in (A), and selecting a plant cell in which a desired gene is introduced into a target position in the DNA by using, as an index, expression of a specific character or disappearance of a specific character observed during the culturing, and
(C) culturing the plant cell selected in (B) to redifferentiate a plant tissue or a plant individual.

14. A method for modifying a DNA, which comprises introducing a vector comprising a first genetic moiety inserted between two site-specific recombinase recognition sequences, and a site-specific recombinase gene which recognizes the site-specific recoinbinase recognition sequences, and a lethal induction gene or a morphological abnormality induction gene which are positioned outside the first genetic moiety, into a host cell other than a human germ cell having a DNA in which one site-specific recombinase recognition sequence is present; and deleting or inverting a part of the host DNA by a site-specific recombination at the one site-specific recombinase recognition sequence on the host DNA.

15. The method for modifying a DNA according to claim 14, wherein the host is a plant.

16. A method for modifying a DNA, which comprises introducing a vector comprising a first genetic moiety inserted between two site-specific recombinase recognition sequences, and a site-specific recombinase gene which recognizes the site-specific recombinase recognition sequences, and a lethal induction gene or a morphological abnormality induction gene which are positioned outside the first genetic moiety, into a host cell other than a human germ cell having a DNA in which a moiety inserted between two site-specific recombinase recognition sequences is present; and deleting or inverting a part of the host DNA by a site-specific recombination at the two site-specific recombinase recognition sequences on the host DNA.

17. The method for modifying a plant DNA according to claim 16, wherein the host is a plant.

18. A method for producing a plant tissue or a plant individual in which a part of a DNA is deleted or inverted, which comprises the following steps (A) to (C):
(A) introducing a vector comprising a first genetic moiety inserted between two site-specific recombinase recognition sequences, a site-specific recombinase gene which recognizes the site-specific recombinase recognition sequences, and a lethal induction gene or a morphological abnormality induction gene which are positioned outside the first genetic moiety, into a plant cell having a DNA in which one site-specific recombinase recognition sequence is present or a DNA in which a moiety inserted between two site-specific recombinase recognition sequences is present,
(B) culturing the plant cell into which the vector is introduced in (A), and selecting a plant cell in which a DNA on a target position is deleted or inverted by using, as an index, expression of a specific character or disappearance of a specific character observed during the culturing, and
(C) culturing the plant cell selected in (B) to redifferentiate a plant tissue or a plant individual.

## Patentansprüche

1. Vektor, der Folgendes umfasst: einen ersten genetischen Teil, der zwischen zwei ortsspezifischen Rekombinase-Erkennungssequenzen, einem ortsspezifischen Rekombinasegen, das die ortsspezifischen Rekombinase-Erkennungssequenzen erkennt, und einem letalen Induktionsgen oder einem Gen zur Induktion einer morphologischen Abnormalität insertiert ist, worin das ortsspezifische Rekombinasegen und das letale Induktionsgen oder das Gen zur Induktion einer morphologischen Abnormalität an der Außenseite des ersten genetischen Teils positioniert ist.

2. Vektor nach Anspruch 1, worin der erste genetische Teil, das ortsspezifische Rekombinasegen und das letale Induktionsgen oder das Gen zur Induktion einer morphologischen Abnormalität dergestalt positioniert sind, dass sie mittels der von dem ortsspezifischen Rekombinasegen exprimierten Rekombinase aus dem Vektor entfernt werden können.

3. Vektor nach Anspruch 1, worin das Gen zur Induktion einer morphologischen Abnormalität ein mit einem Pflanzenhormon verwandtes Gen darstellt.

4. Vektor nach Anspruch 3, worin das mit einem Pflanzenhormon verwandte Gen ein Cytokinin-Synthesegen darstellt.

5. Vektor nach Anspruch 1, worin ein erwünschtes Gen an der Innenseite des ersten genetischen Teils positioniert ist.

6. Vektor nach Anspruch 1, worin ein Teil oder das Ganze eines selektierbaren Markergens an der Innenseite des ersten genetischen Teils positioniert ist.

7. Vektor nach Anspruch 6, worin mindestens ein Promotorteil des selektierbaren Markergens an der Innenseite des ersten genetischen Teils positioniert ist.

8. Vektor nach Anspruch 6, worin mindestens ein Strukturgenteil des selektierbaren Markergens an der Innenseite des ersten genetischen Teils positioniert ist.

9. Verfahren zum Einführen eines Gens, welches Folgendes umfasst: Einführen eines Vektors, umfassend einen ersten genetischen Teil, der zwischen zwei ortsspezifischen Rekombinase-Erkennungsequenzen insertiert ist, und ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Wirtszelle mit Ausnahme einer humanen Keimzelle mit einer DNA, in der eine ortsspezifische Rekombinase-Erkennungssequenz anwesend ist; und Einführen des ersten genetischen Teils in die Wirts-DNA mittels einer ortsspezifischen Rekombination an der einen ortsspezifischen Rekombinase-Erkennungssequenz auf der Wirts-DNA.

10. Verfahren zum Einführen eines Gens in eine Pflanze nach Anspruch 9, worin der Wirt eine Pflanze darstellt.

11. Verfahren zum Einführen eines Gens, welches Folgendes umfasst: Einführen eines Vektors, umfassend einen ersten genetischen Teil, der zwischen zwei ortsspezifischen Rekombinase-Erkennungssequenzen insertiert ist, und ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Wirtszelle mit Ausnahme einer humanen Keimzelle mit einer DNA, in der ein zwischen zwei ortsspezifischen Rekombinase-Erkennungssequenzen insertierter Teil anwesend ist; und Einführen des ersten genetischen Teils in die Wirts-DNA mittels einer ortsspezifischen Rekombination an den zwei ortsspezifischen Rekombinase-Erkennungssequenzen auf der Wirts-DNA.

12. Verfahren zum Einführen eines Gens in eine Pflanze nach Anspruch 11, worin der Wirt eine Pflanze darstellt.

13. Verfahren zum Herstellen eines Pflanzengewebes oder eines Pflazenindividuums, in das ein erwünschtes Gen eingeführt wird, das die folgenden Schritte (A) bis (C) umfasst:
(A) Einführen eines Vektors, der Folgendes umfasst: einen ersten genetischen Teil, der zwischen zwei ortsspezifischen Rekombinase-Erkennungssequenzen insertiert wird, und ein erwünschtes Gen an der Innenseite des ersten genetischen Teils umfasst, und ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Pflanzenzelle mit einer DNA, in der eine ortsspezifische Rekombinase-Erkennungssequenz anwesend ist oder eine DNA, in der ein zwischen zwei ortsspezifischen Rekombinase-Erkennungssequenzen insertierter Teil anwesend ist,
(B) Kultivieren der Pflanzenzelle, in die der Vektor in (A) eingeführt ist, und Auswählen einer Pflanzenzelle, in die ein erwünschtes Gen in eine Zielposition in der DNA unter Verwendung, als ein Anzeichen, der während des Kultivierens beobachteten Expression eines spezifischen Merkmals oder des Verschwindens eines spezifischen Merkmals eingeführt wird, und
(C) Kultivieren der in (B) ausgewählten Pflanzenzelle zur Redifferenzierung eines Pflanzengewebes oder eines Pflanzenindividuums.

14. Verfahren zum Modifizieren einer DNA, das Folgendes umfasst: Einführen eines Vektors, umfassend einen ersten genetischen Teil, der zwischen zwei ortsspezifischen Rekombinase-Erkennungssequenzen insertiert ist, und ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Wirtszelle mit Ausnahme einer humanen Keimzelle mit einer DNA, in der eine ortsspezifische Rekombinase-Erkennungssequenz anwesend ist; und Deletieren oder Invertieren eines Teils der Wirts-DNA mittels einer ortsspezifischen Rekombination an der ortsspezifischen Rekombinase-Erkennungssequenz auf der Wirts-DNA.

15. Verfahren zum Modifizieren einer DNA nach Anspruch 14, worin der Wirt eine Pflanze darstellt.

16. Verfahren zum Modifizieren einer DNA, das Folgendes umfasst: Einführen eines Vektors umfassend einen ersten genetischen Teil, der zwischen zwei ortsspezifischen Rekombinase-Erkennungssequenzen insertiert ist, und ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Wirtszelle mit Ausnahme einer humanen Keimzelle mit einer DNA, in der ein zwischen zwei ortsspezifischen Rekombinase-Erkennungssequenzen insertierter Teil anwesend ist; und Deletieren oder Invertieren eines Teils der Wirts-DNA mittels einer ortsspezifischen Rekombination an den zwei ortsspezifischen Rekombinase-Erkennungssequenzen auf der Wirts-DNA.

17. Verfahren zum Modifizieren einer Pflanzen-DNA nach Anspruch 16, worin der Wirt eine Pflanze darstellt.

18. Verfahren zum Herstellen eines Pflanzengewebes oder eines Pflanzenindividuums, worin ein Teil einer DNA deletiert oder invertiert wird, welches die folgenden Schritte (A) bis (C) umfasst:
(A) Einführen eines Vektors, der Folgendes umfasst: einen ersten genetischen Teil, der zwischen zwei ortsspezifischen Rekombinase-Erkennungssequenzen insertiert ist, ein ortsspezifisches Rekombinasegen, das die ortsspezifischen Rekombinase-Erkennungssequenzen erkennt, und ein letales Induktionsgen oder ein Gen zur Induktion einer morphologischen Abnormalität, die an der Außenseite des ersten genetischen Teils positioniert sind, in eine Pflanzenzelle mit einer DNA, in der eine ortsspezifische Rekombinase-Erkennungssequenz anwesend ist oder eine DNA, in der ein zwischen zwei ortsspezifischen Rekombinase-Erkennungssequenzen insertierter Teil anwesend ist,
(B) Kultivieren der Pflanzenzelle, in die der Vektor in (A) eingeführt ist, und Auswählen einer Pflanzenzelle, in der eine DNA an einer Zielposition unter Verwendung, als ein Anzeichen, der während des Kultivierens beobachteten Expression eines spezifischen Merkmals oder des Verschwindens eines spezifischen Merkmals, deletiert oder invertiert wird, und
(C) Kultivieren der Pflanzenzelle, die in (B) zur Redifferenzierung eines Pflanzengewebes oder eines Pflanzenindividuums ausgewählt ist.

## Revendications

1. Vecteur comprenant une première fraction génétique insérée entre deux séquences de reconnaissance de recombinase site-spécifique, un gène de recombinase site-spécifique qui reconnaît les séquences de reconnaissance de recombinase site-spécifique et un gène d'induction létale ou un gène d'induction d'anomalie morphologique, dans lequel le gène de recombinase site-spécifique et le gène d'induction létale ou le gène d'induction d'anomalie morphologique sont positionnés à l'extérieur de la première fraction génétique.

2. Le vecteur selon la revendication 1, dans lequel la première fraction génétique, le gène de recombinase site-spécifique et le gène d'induction létale ou le gène d'induction d'anomalie morphologique sont positionnés de telle sorte qu'ils peuvent être retirés du vecteur par recombinase exprimée du gène de recombinase site-spécifique.

3. Le vecteur selon la revendication 1, dans lequel le gène d'induction d'anomalie morphologique est un gène associé à une hormone végétale.

4. Le vecteur selon la revendication 3, dans lequel le gène associé à une hormone végétale est un gène de synthèse des cytokinines.

5. Le vecteur selon la revendication 1, dans lequel un gène désiré est positionné à l'intérieur de la première fraction génétique.

6. Le vecteur selon la revendication 1, dans lequel une partie ou l'ensemble d'un gène marqueur sélectionnable est positionné à l'intérieur de la première fraction génétique.

7. Le vecteur selon la revendication 6, dans lequel au moins une fraction promoteur du gène marqueur sélectionnable est positionnée à l'intérieur de la première fraction génétique.

8. Le vecteur selon la revendication 6, dans lequel au moins une fraction génique structurelle du gène marqueur sélectionnable est positionnée à l'intérieur de la première fraction génétique.

9. Procédé d'introduction d'un gène, lequel comprend introduire un vecteur comprenant une première fraction génétique insérée entre deux séquences de reconnaissance de recombinase site-spécifique et un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule hôte autre qu'une cellule germinale humaine ayant un ADN dans lequel une séquence de reconnaissance de recombinase site-spécifique est présente; et introduire la première fraction génétique dans l'ADN hôte par une recombinaison site-spécifique au niveau de la séquence de reconnaissance de recombinase site-spécifique sur l'ADN hôte.

10. Le procédé d'introduction d'un gène dans une plante selon la revendication 9, où l'hôte est une plante.

11. Procédé d'introduction d'un gène, lequel comprend introduire un vecteur comprenant une première fraction génétique insérée entre deux séquences de reconnaissance de recombinase site-spécifique et un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule hôte autre qu'une cellule germinale humaine ayant un ADN dans lequel une fraction insérée entre deux séquences de reconnaissance de recombinase site-spécifique est présente; et introduire la première fraction génétique dans l'ADN hôte par une recombinaison site-spécifique au niveau des deux séquences de reconnaissance de recombinase site-spécifique sur l'ADN hôte.

12. Le procédé d'introduction d'un gène dans une plante selon la revendication 11, dans lequel l'hôte est une plante.

13. Procédé de production d'un tissu végétal ou d'une plante particulière dans lequel/laquelle un gène désiré est introduit, lequel comprend les étapes (A) à (C) suivantes:
(A) introduire un vecteur comprenant une première fraction génétique insérée entre deux séquences de reconnaissance de recombinase site-spécifique et comprenant un gène désiré à l'intérieur de la première fraction génétique, et un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule végétale ayant un ADN dans lequel une séquence de reconnaissance de recombinase site-spécifique est présente ou un ADN dans lequel une fraction insérée entre deux séquences de reconnaissance de recombinase site-spécifique est présente,
(B) cultiver la cellule végétale dans laquelle le vecteur est introduit dans (A) et sélectionner une cellule végétale dans laquelle un gène désiré est introduit à une position cible dans l'ADN en utilisant, en tant qu'indice, l'expression d'un caractère spécifique ou la disparition d'un caractère spécifique observé durant la culture, et
(C) cultiver la cellule végétale sélectionnée dans (B) pour redifférencier un tissu végétal ou une plante particulière.

14. Procédé de modification d'un ADN, lequel comprend introduire un vecteur comprenant une première fraction génétique insérée entre deux séquences de reconnaissance de recombinase site-spécifique et un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule hôte autre qu'une cellule germinale humaine ayant un ADN dans lequel une séquence de reconnaissance de recombinase site-spécifique est présente; et supprimer ou inverser une partie de l'ADN hôte par une recombinaison site-spécifique au niveau de la séquence de reconnaissance de recombinase site-spécifique sur l'ADN hôte.

15. Le procédé de modification d'un ADN selon la revendication 14, dans lequel l'hôte est une plante.

16. Procédé de modification d'un ADN, lequel comprend introduire un vecteur comprenant une première fraction génétique insérée entre deux séquences de reconnaissance de recombinase site-spécifique et un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule hôte autre qu'une cellule germinale humaine ayant un ADN dans lequel une fraction insérée entre deux séquences de reconnaissance de recombinase site-spécifique est présente; et supprimer ou inverser une partie de l'ADN hôte par une recombinaison site-spécifique au niveau des deux séquences de reconnaissance de recombinase site-spécifique sur l'ADN hôte.

17. Le procédé de modification d'un ADN végétal selon la revendication 16, dans lequel l'hôte est une plante.

18. Procédé de production d'un tissu végétal ou d'une plante particulière dans lequel/laquelle une partie d'un ADN est supprimée ou inversée, lequel comprend les étapes (A) à (C) suivantes:
(A) introduire un vecteur comprenant une première fraction génétique insérée entre deux séquences de reconnaissance de recombinase site-spécifique, un gène de recombinase site-spécifique qui reconnaît les séquences de reconnaissance de recombinase site-spécifique et un gène d'induction létale ou un gène d'induction d'anomalie morphologique qui sont positionnés à l'extérieur de la première fraction génétique, dans une cellule végétale ayant un ADN dans lequel une séquence de reconnaissance de recombinase site-spécifique est présente ou un ADN dans lequel une fraction insérée entre deux séquences de reconnaissance de recombinase site-spécifique est présente,
(B) cultiver la cellule végétale dans laquelle le vecteur est introduit dans (A) et sélectionner une cellule végétale dans laquelle un ADN à une position cible est supprimé ou inversé en utilisant, en tant qu'indice, l'expression d'un caractère spécifique ou la disparition d'un caractère spécifique observé durant la culture, et
(C) cultiver la cellule végétale sélectionnée dans (B) pour redifférencier un tissu végétal ou une plante particulière.
